# EUROPEAN PATENT APPLICATION

(11) **EP 4 356 890 A1**
(43) Date of publication of application: **24.04.2024**
(21) Application number: 21950994.0
(22) Date of filing: 13.12.2021
(51) Int. Cl.: A61F 13/49, A61F 13/496, A61F 13/51

(54) **UNDERPANTS-TYPE ABSORBENT ARTICLE**

(30) Priority: 20.07.2021 JP 2021119911
(71) Applicant: Unicharm Corporation, Ehime 799-0111 (JP)
(72) Inventor: NAGAI, Takahito, Kanonji-shi, Kagawa 769-1602 (JP); MUKAI, Hirotomo, Kanonji-shi, Kagawa 769-1602 (JP); OHTSUBO, Toshifumi, Kanonji-shi, Kagawa 769-1602 (JP); AKINO, Chieri, Kanonji-shi, Kagawa 769-1602 (JP)
(74) Representative: karo IP
(86) International application number: PCT/JP2021/045853
(87) International publication number: WO 2023/002645

(57) **Abstract**

A pants-type absorbent article (1) comprising an absorbent body (10) and a belt portion (30) connecting between a front-side upper end (10eLf) and a back-side upper end (10eLb) of the absorbent body (10), wherein the belt portion (30) is joined with the absorbent body (10) via a joint part (40) that slopes from an inner side toward an outer side in the right-left direction and from a top side toward a bottom side in the vertical direction starting at a beltline opening (BH) toward leg-side openings (LH), and, when said pants-type absorbent article (1) in an extended state is viewed in the front-back direction, the joint part (40) includes a portion (40fp) that projects inward in the right-left direction as compared with a straight line connecting an upper end (40fea) and a lower end (40feb) in the vertical direction of the joint part (40).

## Description

### FIELD

The present invention concerns underpants-shaped absorbent article.

### BACKGROUND

Conventionally, there are widely known underpants-shaped absorbent articles in which a front waist portion and a back waist portion, both of which have stretchability, are connected by side joining portions provided in two lateral ends, thereby forming a waist opening and a pair of leg openings. For example, Patent Document 1 discloses an underpants-shaped disposable article in which waist elastic members are sandwiched between two sheet members, and the elastic members are fixed to the sheet members by three or more stripe-shaped side joining portions provided in a direction that intersects the stretching/contracting direction of the elastic members.

### [CITATION LIST]

### [PATENT LITERATURE]

[Patent Document 1] WO2018/092559

### SUMMARY

### [TECHNICAL PROBLEM]

After putting on such an underpants-shaped absorbent article, when removing it from the wearer's body (taking off the underpants-shaped absorbent article which is in a put-on state), it is common to spread open the waist opening by tearing the side joining portions. However, since the waist portions themselves have stretchability in the lateral direction, the force for tearing the side joining portions may be used to laterally stretch the waist portions, or it may become difficult for the force to act on the side joining portions, making the side joining portions difficult to tear. This causes a problem that the underpants-shaped absorbent article is difficult to remove from the wearer's body.

The present invention was achieved in light of conventional problems such as that described above, and an aspect of the present invention is to facilitate an operation of removing from the wearer's body an underpants-shaped absorbent article in which a front waist portion and a back waist portion, both of which have stretchability, are connected by side joining portions, the removing being made by tearing the side joining portion.

### [SOLUTION TO PROBLEM]

A main aspect of the present invention of achieving the above-described aspect is an underpants-shaped absorbent article having a vertical direction and a lateral direction that intersect with each other, the underpants-shaped absorbent article including: an absorbent main body; and a waist portion in which a front waist portion and a back waist portion are joined to each other by a pair of side joining portions in two ends in the lateral direction; the waist portion having a stretchability in the lateral direction, in at least a portion located between the pair of side joining portions, the waist portion that is in a stretched state having: inner end regions that are located above an upper end of the absorbent main body in the vertical direction and within a range of 20 mm inward from an inner end of the side joining portion in the lateral direction; and a central region that is located above the upper end of the absorbent main body in the vertical direction and is sandwiched between a pair of the inner end regions in the lateral direction, concerning a difference between a lateral length of the inner end region in the stretched state and a lateral length of the inner end region in a natural state, concerning a difference between a lateral length of the central region in the stretched state and a lateral length of the central region in the natural state, a value obtained by dividing the difference of the inner end region by the lateral length of the inner end region in the stretched state being smaller than a value obtained by dividing the difference of the central region by the lateral length of the central region in the stretched state.

Features of the present invention other than the above will become clear by reading the description of the present specification with reference to the accompanying drawings.

### [ADVANTAGEOUS EFFECT OF THE INVENTION]

According to the present invention, it is possible to facilitate an operation of removing from the wearer's body an underpants-shaped absorbent article in which a front waist portion and a back waist portion, both of which have stretchability, are connected by side joining portions, the removing being made by tearing the side joining portion.

### BRIEF DESCRIPTION OF DRAWINGS

FIG. 1 is a schematic perspective view of a diaper 1.
FIG. 2 is a schematic plan view of the diaper 1 in an unfolded and stretched state when viewed from a wearer's skin side.
FIG. 3 is a sectional view taken along line A-A in FIG. 2, and also a sectional view taken along line B-B in FIG. 2.
FIG. 4 is a schematic plan view of a front waist portion 31 in the unfolded and stretched state when viewed from a non-skin side.
FIG. 5A and 5B are diagrams showing a function to attach an elastic member 35, and are enlarged schematic view of the portion X in FIG. 4.
FIG. 6 is a diagram illustrating the shape and arrangement of welded portions 50, and is an enlarged schematic diagram in the portion C in FIG. 4.
FIG. 7 is a diagram illustrating the configuration of a side joining portion 70.
FIG. 8 is a schematic plan view showing a partial perspective view of how the diaper 1 is manufactured in the production line 100.
FIG. 9 is a diagram illustrating a processing performed at the first machining position PK1.
FIG. 10 is a diagram illustrating the stretchability of the front waist portion 31 (41) in conventional underpants-shaped diapers as a comparative example.
FIG. 11 is a diagram illustrating the stretchability of the front waist portion 31 (41) in the diaper 1 of the first embodiment.
FIG. 12 is a diagram illustrating stretchability in an absorbent-body side region SA of the front waist portion 31 (41).
FIG. 13 is a schematic plan view illustrating a modified example of a back waist portion 41.
FIG. 14 is a diagram illustrating the shape and arrangement of welded portions 50 of the second embodiment.

### DESCRIPTION OF EMBODIMENTS

At least the following matters will be clear with the description of this specification and the attached drawings.

An underpants-shaped absorbent article having a vertical direction and a lateral direction that intersect with each other, the underpants-shaped absorbent article including: an absorbent main body; and a waist portion in which a front waist portion and a back waist portion are joined to each other by a pair of side joining portions in two ends in the lateral direction; the waist portion having a stretchability in the lateral direction, in at least a portion located between the pair of side joining portions, the waist portion that is in a stretched state having: inner end regions that are located above an upper end of the absorbent main body in the vertical direction and within a range of 20 mm inward from an inner end of the side joining portion in the lateral direction; and a central region that is located above the upper end of the absorbent main body in the vertical direction and is sandwiched between a pair of the inner end regions in the lateral direction, concerning a difference between a lateral length of the inner end region in the stretched state and a lateral length of the inner end region in a natural state, concerning a difference between a lateral length of the central region in the stretched state and a lateral length of the central region in the natural state, a value obtained by dividing the difference of the inner end region by the lateral length of the inner end region in the stretched state being smaller than a value obtained by dividing the difference of the central region by the lateral length of the central region in the stretched state.

According to such an underpants-shaped absorbent article, the stretchability in the inner end region is lower than the stretchability in the central region. Accordingly, when attempting to tear the side joining portion during the attachment/detachment operation, the waist portion is less likely to stretch/contract in the lateral direction in the inner end region, which is located near the side joining portion. This makes a force likely to efficiently act on the side joining portion, making it possible to easily tear the side joining portion. This can make it easier to do the operation of removing the underpants-shaped absorbent article from the wearer's body.

In such an underpants-shaped absorbent article, it is desirable that the waist portion has an outer end region that is located outside the inner end of the side joining portion in the lateral direction, and that concerning a difference between a lateral length of the outer end region in the stretched state and a lateral length of the outer end region in the natural state, concerning the difference between a lateral length of the inner end region in the stretched state and the lateral length of the inner end region in the natural state, a value obtained by dividing the difference of the outer end region by the lateral length of the outer end region in the stretched state is smaller than a value obtained by dividing the difference of the inner end region by the lateral length of the inner end region in the stretched state.

According to such an underpants-shaped absorbent article, it reduces lateral stretching/contracting in the outer end region, which includes the side joining portion, and this makes the force that will tear the side joining portion more likely to act. Furthermore, the outer end regions are less likely to stretch/contract, making it easier to hold those regions with hands. In addition, it makes it more likely to transmit the force that holds the outer end region and pulls the front waist portion and the back waist portion respectively to the opposite sides, and makes it easier insert fingers into the inner end regions. This makes is possible to easily tear the side joining portion.

In such an underpants-shaped absorbent article, it is desirable that the waist portion has a skin-side sheet and a non-skin-side sheet that are stacked in a thickness direction, that a plurality of elastic members are arranged spacing in the vertical direction between the skin-side sheet and the non-skin-side sheet that are joined by a plurality of welded portions, the plurality of elastic members being capable of stretching and contracting in the lateral direction, that each of the elastic members is attached, in a state of contracting in the lateral direction, to the skin-side sheet and the non-skin-side sheet by being sandwiched between two vertically adjacent ones of the plurality of welded portions, that concerning a difference between a lateral length of an elastic member in the stretched state and a lateral length of the same elastic member in the natural state, a value obtained by dividing the difference of the elastic member that is attached by the welded portion located in the inner end region by the lateral length of the same elastic member in the stretched state is smaller than a value obtained by dividing the difference of the elastic member that is attached by the welded portion located in the central region by the lateral length of the same elastic member in the stretched state.

According to such an underpants-shaped absorbent article, the stretchability of the elastic members attached to the waist portion by the welded portions in the inner end region is lower than the stretchability of the elastic members attached to the waist portion by the welded portions in the central region. This makes the waist portion less likely to stretch/contract in the lateral direction in the inner end region, which is located near the side joining portion. This makes a force likely to efficiently act on the side joining portion, making it possible to easily tear the side joining portion.

In such an underpants-shaped absorbent article, it is desirable that, in the stretched state, a lateral width of at least one of the welded portion located in the inner end region is greater than a minimum value of a lateral width of the welded portion located in the central region.

According to such an underpants-shaped absorbent article, in the inner end region, a wide lateral range of the elastic member is sandwiched with a pair of welded portions (welded portion pair). This makes the waist elastic members less likely to stretch/contract in the lateral direction compared to in the central region, and prevents the inner end regions from stretching excessively. This makes a force likely to act on the side joining portion, making it possible to easily tear the side joining portion.

In such an underpants-shaped absorbent article, it is desirable that, in the stretched state, a value obtained by dividing a sum of a lateral width of the welded portion located in the inner end region by a lateral width of the inner end region is greater than a value obtained by dividing a sum of a lateral width of the welded portion located in the central region by a lateral width of the central region.

According to such an underpants-shaped absorbent article, the stretchability in the inner end region is weakened compared to in the central region, and this prevents the inner end regions from stretching excessively. This makes a force likely to efficiently act on the side joining portion, making it possible to easily tear the side joining portion.

In such an underpants-shaped absorbent article, it is desirable that a lateral length of the welded portion that is located in the lateral direction nearest to an inner end of the side joining portion is longer than a lateral length of at least one of other welded portion that is located inside the welded portion that is located in the lateral direction nearest to an inner end of the side joining portion.

According to such an underpants-shaped absorbent article, it is possible to reduce lateral stretching/contracting of the elastic member in a region within the inner end region that is nearest to the side joining portion. This makes a force likely to more efficiently act on the side joining portion, making it possible to more easily tear the side joining portion.

In such an underpants-shaped absorbent article, it is desirable that the back waist portion has a buttocks cover below a lower end of the side joining portion, at least some of the elastic members is arranged in the buttocks cover, and that a lateral length of the welded portion that is located in the lateral direction nearest to an outer edge portion of the buttocks cover is longer than a lateral length of at least one of other welded portion that is located inside the welded portion that is located in the lateral direction nearest to an inner end of the side joining portion.

According to such an underpants-shaped absorbent article, the width of the welded portions in regions close to the outer edge portion of the buttocks cover is made wider to make the elastic members less likely to stretch/contract, and accordingly it makes a force likely to act on the lower end portion of the side joining portion, making it possible to easily tear the side joining portion from the lower side to the upper side.

In such an underpants-shaped absorbent article, it is desirable that the back waist portion has a buttocks cover below a lower end of the side joining portion, that a leg elastic member that stretches/contracts along an outer edge portion of the buttocks cover is provided, and that the leg elastic member is attached to the back waist portion with adhesive.

According to such an underpants-shaped absorbent article, since the leg elastic member is attached by the adhesive portion, it increases the stiffness of the region extending along the outer edge portion of the buttocks cover. Therefore, when performing an operation of tearing the side joining portion from the lower side to the upper side, even if the buttocks cover is pulled strongly, the buttocks cover is less likely to tear, making it possible to easily tear the side joining portion.

In such an underpants-shaped absorbent article, it is desirable that at least one of the skin-side sheet and the non-skin-side sheet is a spunbond nonwoven fabric sheet in which heat-fusing fibers are joined by a plurality of dispersed heat fused portions, and in the inner end region, either one of the skin-side sheet or the non-skin-side sheet has a portion that overlaps the welded portion and the heat fused portion when seen in the thickness direction.

According to underpants-shaped absorbent article, in the portion where the heat fused portion and the welded portion overlap, protrusions/recesses in the thickness direction are deeper, making fingers likely to become caught compared to other regions. This makes a user easier to hold the inner end regions, making it possible to easily conduct an operation of tearing the side joining portion.

In such an underpants-shaped absorbent article, it is desirable that each of the side joining portions has a plurality of lock welded portions lined up spacing in the vertical direction, and that a depth of the lock welded portion in the thickness direction is deeper than a depth of the welded portion in the thickness direction.

According to underpants-shaped absorbent article, due to the difference in depth between the lock welded portion and the welded portion, when a user holds the waist portion, the user can feel the difference in touch between side joining portion and the remaining portion. This makes the user possible to recognize the portion of the waist portion where the side joining portion is formed, without making a mistake, making it easier to facilitate an operation of holding the inner end region and the outer end region and tearing the side joining portion.

In such an underpants-shaped absorbent article, it is desirable that a maximum value of a lateral length of the lock welded portions is smaller than a maximum value of a lateral length of the welded portion that is provided in the inner end region.

According to such an underpants-shaped absorbent article, compared to the case where the maximum value of the width of the welded portion provided in the inner end region is smaller than the maximum value of the width of the lock welded portions, it is likely to suppress the stretching/contracting of the elastic members in the inner end region. This makes a force likely to act on the side joining portion when conducting the attachment/detachment operation, making it possible to easily tear the side joining portion.

In such an underpants-shaped absorbent article, it is desirable that the waist portion includes: a first elastic member that is capable of stretching and contracting in the lateral direction, and a second elastic member that is located adjacent to the first elastic member in the vertical direction, that a plurality of wrinkles along vertical direction are formed by protruding deforming of a sheet member in the thickness direction, the sheet member being one that constitutes the waist portion, and that at least some of the plurality of wrinkles are continuous between the first elastic member and the second elastic member in the vertical direction.

According to underpants-shaped absorbent article, a continuous wrinkle is formed between the first elastic member and the second elastic member in the vertical direction, so that when the wearer holds the waist portion and pulls it in the lateral direction, it makes the fingers likely to become caught in the wrinkles and less likely to slip. This can make it easier to stably perform the operation of tearing the side joining portion.

In such an underpants-shaped absorbent article, it is desirable that the absorbent main body includes a liquid-absorbent absorbent core, that the waist portion that is in the stretched state has: absorbent-body side regions that are located below the upper end of the absorbent main body in the vertical direction and within a range of 20 mm outward from both ends of the absorbent core in the lateral direction; and leg-circumferential regions that are located below the upper end of the absorbent main body in the vertical direction and are respectively sandwiched between corresponding pairs of the absorbent-body side region and the side joining portion in the lateral direction, and that concerning a difference between a lateral length of the absorbent-body side region in the stretched state and a lateral length of the absorbent-body side region in the natural state, concerning a difference between a lateral length of the leg-circumferential region in the stretched state and a lateral length of the leg-circumferential region in the natural state, a value obtained by dividing the difference of the absorbent-body side region by the lateral length of the absorbent-body side region in the stretched state is smaller than a value obtained by dividing the difference of the leg-circumferential region by the lateral length of the leg-circumferential region in the stretched state.

According to such an underpants-shaped absorbent article, the dimensional change ratio of the absorbent-body side region is small and the absorbent-body side region is less likely to stretch/contract in the lateral direction. Therefore, when the absorbent-body side region is pulled upward at the time of putting on the absorbent article, the absorbent core is also likely to be pulled upward. This makes it possible to enhance the fit of the absorbent core in the crotch portion.

In addition, an underpants-shaped absorbent article having a vertical direction and a lateral direction that intersect with each other, underpants-shaped absorbent article including: an absorbent main body having a liquid-absorbent absorbent core; a waist portion in which a front waist portion and a back waist portion are joined to each other by a pair of side joining portions in two ends in the lateral direction; the waist portion having a stretchability in the lateral direction, in at least a portion located between the pair of side joining portions, the waist portion that is in a stretched state having: an absorbent-body side region that is located below an upper end of the absorbent main body in the vertical direction and within a range of 20 mm outward from two ends of the absorbent core in the lateral direction, and a leg-circumferential region that is located below the upper end of the absorbent main body in the vertical direction and is sandwiched between the absorbent-body side region and the side joining portion in the lateral direction, concerning a difference between a lateral length of the absorbent-body side region in the stretched state and a lateral length of the absorbent-body side region in a natural state, concerning a difference between a lateral length of the leg-circumferential region in the stretched state and a lateral length of the leg-circumferential region in the natural state, a value obtained by dividing the difference of the absorbent-body side region by the lateral length of the absorbent-body side region in the stretched state being smaller than a value obtained by dividing the difference of the leg-circumferential region by the lateral length of the leg-circumferential region in the stretched state.

According to such an underpants-shaped absorbent article, the dimensional change ratio of the absorbent-body side region is small and the absorbent-body side region is less likely to stretch/contract in the lateral direction. Therefore, when the absorbent-body side region is pulled upward at the time of putting on the absorbent article, the absorbent core is also likely to be pulled upward. This makes it possible to enhance the fit of the absorbent core in the crotch portion.

### First embodiment

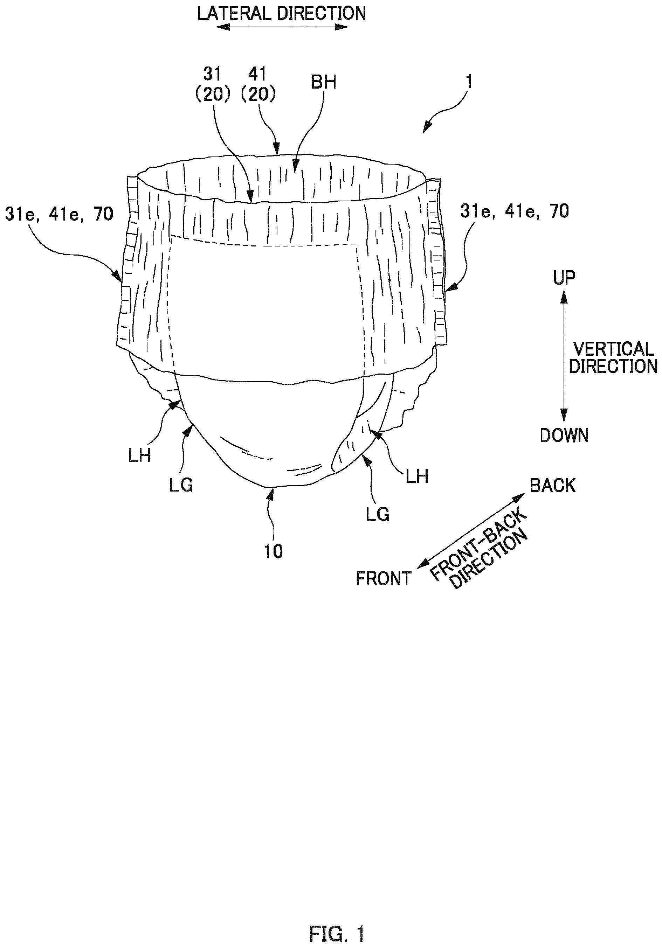

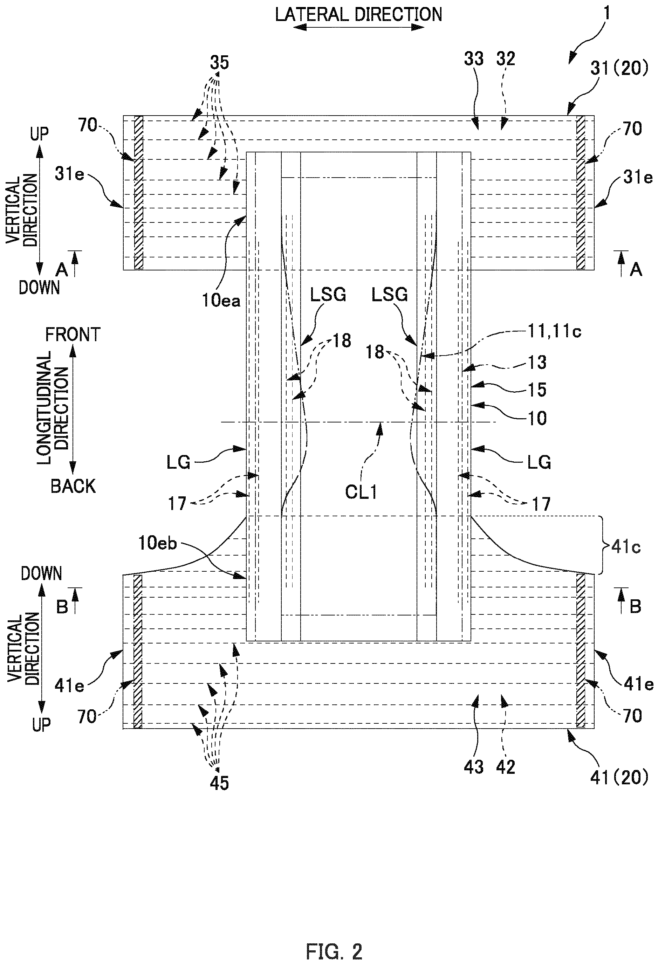

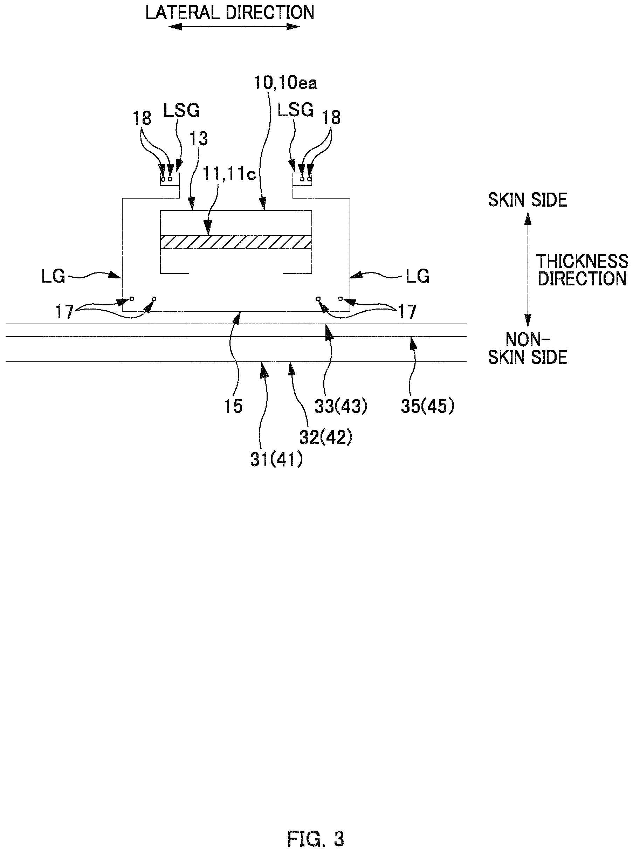

Basic configuration of underpants-shaped disposable diaper An underpants-shaped disposable diaper 1 (hereinafter also referred to as "diaper 1") will be described by way of example of underpants-shaped absorbent article according to the first embodiment. FIG. 1 is a schematic perspective view of the diaper 1. FIG. 2 is a schematic plan view of the diaper 1 in an unfolded and stretched state when viewed from a wearer's skin side. FIG. 3 is a sectional view taken along line A-A in FIG. 2, and also a sectional view taken along line B-B in FIG. 2.

This diaper 1 in an underpants-shaped state before wearing as shown in FIG. 1 has three directions that intersect with each other, namely a "vertical direction", a "lateral direction", and a "front-back direction". With respect to the vertical direction, the upper side corresponds to the waist opening side of the wearer, and the lower side corresponds to the crotch side of the wearer. Further, with respect to the front-back direction, the front side corresponds to the stomach side of the wearer, and the back side corresponds to the back side of the wearer.

In the underpants-shaped state in FIG. 1, the diaper 1 includes an absorbent main body 10 and a waist portion 20. The waist portion 20 includes: a front waist portion 31 which has stretchability along the lateral direction; and a back waist portion 41 which has stretchability in the lateral direction, and which is located on the back side of the front waist portion 31 and forms the waist opening BH together with the front waist portion 31 in the upper side in the vertical direction. The absorbent main body 10 is provided as a crotch portion, between front waist portion 31 and back waist portion 41.

Further, the lateral end portions 31e and 31e in the front waist portion 31 and the lateral end portions 41e and 41e in the back waist portion 41 are respectively joined in the side joining portions 70, which serve as welded portions. Thus, the front waist portion 31 and the back waist portion 41 cooperate with the absorbent main body 10 to form leg openings LH and LH in the lower side and on both lateral sides.

Here, the "unfolded state" refers the following state: the front waist portion 31 and the back waist portion 41 are separated by releasing the joining of the aforementioned side joining portions 70 that are provided in the diaper 1 in the underpants-shaped state shown in FIG. 1 on both lateral sides, and in addition the diaper 1 is unfolded on a flat surface by spreading open in the vertical direction. In addition, the "stretched state" refers to the state in which the product (diaper 1) has been stretched to the extent that wrinkles are eliminated, or more specifically, the state in which the diaper 1 is stretched such that the dimensions of constituent members of the diaper 1 (e.g., the absorbent main body 10, the front waist portion 31, etc.) match or are closer to the dimensions of the members on their own.

In the unfolded state, the diaper 1 has three directions that intersect with each other: a "longitudinal direction," a "lateral direction," and a "thickness direction (the direction passing through the paper surface in FIG. 2)". Note that the longitudinal direction conforms to the vertical direction mentioned above. The one side in the longitudinal direction corresponds to the front side, and the other side corresponds to the back side. Further, the outside in the longitudinal direction corresponds to the upper side in the vertical direction (waist opening side), and the inside in the longitudinal direction corresponds to the lower side (crotch side) in the vertical direction. Since the longitudinal direction and the vertical direction are directions that resemble each other, for the sake of convenience hereinafter, the vertical direction will sometimes be used in place of the longitudinal direction in the unfolded state as well. Furthermore, the lateral direction is synonymous with the lateral direction in the previously described underpants-shaped state. Further, with respect to the thickness direction, one side corresponds to the skin side that comes into contact with the wearer's body, and the other side corresponds to the opposite non-skin side.

In the unfolded state shown in FIG. 2, the front waist portion 31 is arranged extending in the lateral direction, and the back waist portion 41 is arranged extending in the lateral direction at a position that is separated from the front waist portion 31 by a predetermined distance in the longitudinal direction. The absorbent main body 10 spans along the longitudinal direction between the front waist portion 31 and the back waist portion 41, and the longitudinal end portions 10ea and 10eb of the absorbent main body 10 (i.e., the front upper end portion 10ea and the back end portion 10eb in the underpants-shaped state) are respectively joined and fixed to the closest waist portions 31 and 41, thus forming a substantially H-like outer shape in a plan view. The diaper 1 in this state is folded one time at a folding position that is at the predetermined longitudinal position CL1 of the absorbent main body 10 (corresponding to the longitudinal central position of the diaper 1), and the lateral end portions 31e and 41e of the waist portions 31 and 41, which face each other in the folded state, are joined to each other in the above-mentioned side joining portions 70. Accordingly, the waist portions 31 and 41 become connected to each other in a ring shape, thus obtaining the diaper 1 in the underpants-shaped state in which the waist opening BH and a pair of leg openings LH and LH are formed as shown in FIG. 1.

### Absorbent main body 10

The absorbent main body 10 has a substantially rectangular shape in a plan view in the unfolded state in FIG. 2. The longitudinal direction of the absorbent main body 10 conforms to the longitudinal direction of the diaper 1. Also, as shown in FIG. 3, the absorbent main body 10 includes: an absorbent body 11; a liquid-permeable top sheet 13 that covers the absorbent body 11 from the skin side and forms the skin-side surface of the absorbent main body 10; and a liquid-impermeable back sheet 15 that covers the absorbent body 11 from the non-skin side and forms the non-skin-side surface of the absorbent main body 10.

The absorbent body 11 has a liquid-absorbent absorbent core 11c, and a core-wrapping sheet (not shown) that covers the outer circumferential surface of the core 11c. The absorbent core 11c is a molded body that is made of a liquid absorbent material, such as pulp fiber and superabsorbent polymer, into a substantially hourglass shape in a plan view, as one example of a predetermined shape. The core-wrapping sheet can made of a liquid-permeable sheet such as tissue paper or nonwoven fabric, but the core-wrapping sheet is not required to be provided. Further, the absorbent core 11c is not limited to having the above-described substantially hourglass shape in a plan view, and may have any other shape.

The top sheet 13 is a liquid-permeable soft sheet made of nonwoven fabric or the like. The back sheet 15 is also a liquid-impermeable soft sheet. One example of the back sheet 15 is a two-layer structure laminate sheet including: a liquid-impermeable leak-proof sheet made of a polyethylene film or a polypropylene film; and an exterior sheet that is made of nonwoven fabric and is affixed to the non-skin side of the leak-proof sheet.

As shown in FIG. 2, at least the back sheet 15 is a sheet having a planar size according to which it projects from the absorbent body 11 in the longitudinal direction and the lateral direction. Leg gathers LG that stretch and contract in the longitudinal direction are formed in the portions that protrude in the lateral direction. Specifically, elastic strings 17 that serve as elastic members and extend in the longitudinal direction are fixed in the protruding portion in a state of being stretched in the longitudinal direction, thus forming the stretchable leg gathers LG in these portions.

Further, as shown in FIGS. 2 and 3, the absorbent main body 10 has barrier cuffs LSG as leak-proof wall portions in the lateral end portions for the purpose of preventing side leakage. Specifically, in the lateral end portions of the absorbent main body 10, a configuration is provided in which elastic strings 18 serving as elastic members and extending in the longitudinal direction are attached, in a state of being stretched in the longitudinal direction, to sheet-like portions that will form the barrier cuffs LSG.

### Waist portions 31 and 41

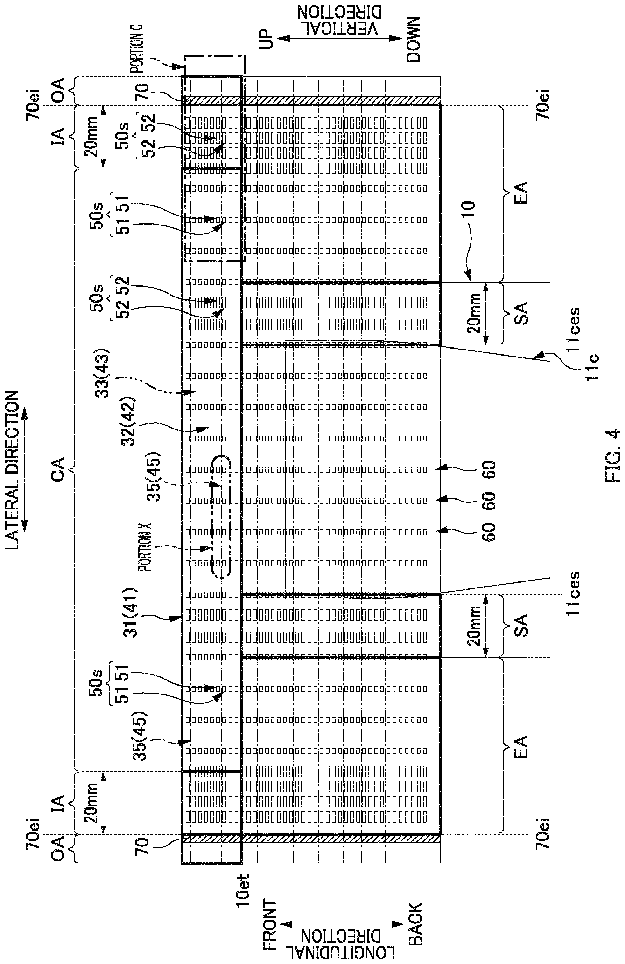

As shown in FIG. 2, the front waist portion 31 is a sheet member that has a substantially rectangular shape in a plan view and is constituted by a first sheet 32 and a second sheet 33 which are stacked in the thickness direction. Specifically, as shown in FIG. 3, the first sheet 32 and the second sheet 33 are overlaid in the thickness direction. And, a pair of facing surfaces that faces each other are joined to each other by a plurality of welded portions 50, 50, ... that are arranged discretely spacing in the vertical direction and the lateral direction H, as shown in later-described FIG. 4. As shown in FIG. 2, the front waist portion 31 is arranged so as to protrude out from the absorbent main body 10 on two lateral sides, and is overlaid on and joined to the non-skin side of the front end portion (front upper end portion) 10ea of the absorbent main body 10.

Similarly to the front waist portion 31, the back waist portion 41 is a sheet member that has a substantially rectangular shape in a plan view and is constituted by a first sheet 42 and a second sheet 43 which are stacked in the thickness direction. Specifically, as shown in FIG. 3, the first sheet 32 and the second sheet 33 are overlaid in the thickness direction. And a pair of facing surfaces that faces each other are joined to each other by a plurality of welded portions 50, 50, ... (corresponding to joining portions) that are arranged discretely spacing in the vertical direction (longitudinal direction) and the lateral direction, similar to the case of the front waist portion 31 in FIG. 4. As shown in FIG. 2, the back waist portion 41 is arranged so as to protrude out from the absorbent main body 10 on two lateral sides, and is overlaid on and joined to the non-skin side of the back end portion (back upper end portion) 10eb of the absorbent main body 10.

Note that, the back waist portion 41 has a substantially trapezoidal buttocks cover 41c as shown in FIG. 2, in the vertical direction below the portion where the side joining portion 70 is provided. The buttocks cover 41c is a portion that broadly covers the wearer's buttocks when the diaper 1 is put on.

Further, in this example, spunbond nonwoven fabric is used for both of the first sheet 32 (42) and the second sheet 33 (43) pertaining to the front waist portion 31 (41). However, there is no limitation whatsoever to this, and it is possible to use various other types of nonwoven fabric such as SMS (spunbond/meltblown/spunbond) nonwoven fabric. Further, in this example, standalone fibers made of polypropylene (PP), which is a representative example of thermoplastic, may be used as the constituent fibers of the nonwoven fabric, but there is no limitation whatsoever to this. For example, standalone fibers made of another thermoplastic resin such as polyethylene (PE) may be used, and composite fibers that have a sheath/core structure and are made of PE and PP or the like may be used.

### Specific configuration of front waist portion 31 (41)

The following describes a specific configuration of the front waist portion 31 and the back waist portion 41. As mentioned above, the front waist portion 31 and the back waist portion 41 of the present embodiment have substantially the same configuration. Therefore, when the contents described below is the same for both the front waist portion 31 and the back waist portion 41, only the front waist portion 31 will be described as a representative for both. Regarding back waist portion 41, the reference signs of its components and the like corresponding to those of the front waist portion 31 will be indicated by blanketing as necessary, and the concrete description will be omitted.

FIG. 4 is a schematic plan view of the front waist portion 31 (stretchy sheet) in the unfolded and stretched state when viewed from the non-skin side. In addition, in FIG. 4, the front waist portion 31 (41) is shown as a constituent member of the diaper 1, but the waist portions 31 and 41 can also be applied to uses other than the diaper 1 as a stretchy sheet.

As shown in FIG. 4, in the first sheet 32 (42) and the second sheet 33 (43) pertaining to the front waist portion 31 (41), a plurality of waist elastic members 35, 35,... (45, 45,...), which are elastic strings etc. that can stretch and contract in the lateral direction, are inserted side-by-side spacing in the vertical direction between a pair of facing surfaces that face each other. And the waist elastic members 35, 35, ... (45, 45,...) are attached to the sheets 32 and 33 (42 and 43) with use of the above-mentioned welded portions 50, 50,.... Accordingly, the front waist portion 31 (41) is given stretchability in the lateral direction. Also, the above-mentioned welded portions 50, 50... not only have a function of joining the pair of facing surfaces of the first sheet 32 (42) and the second sheet 33 (43) to each other, but also have a function of attaching the waist elastic members 35 (45) to the first sheet 32 (42) and the second sheet 33 (43).

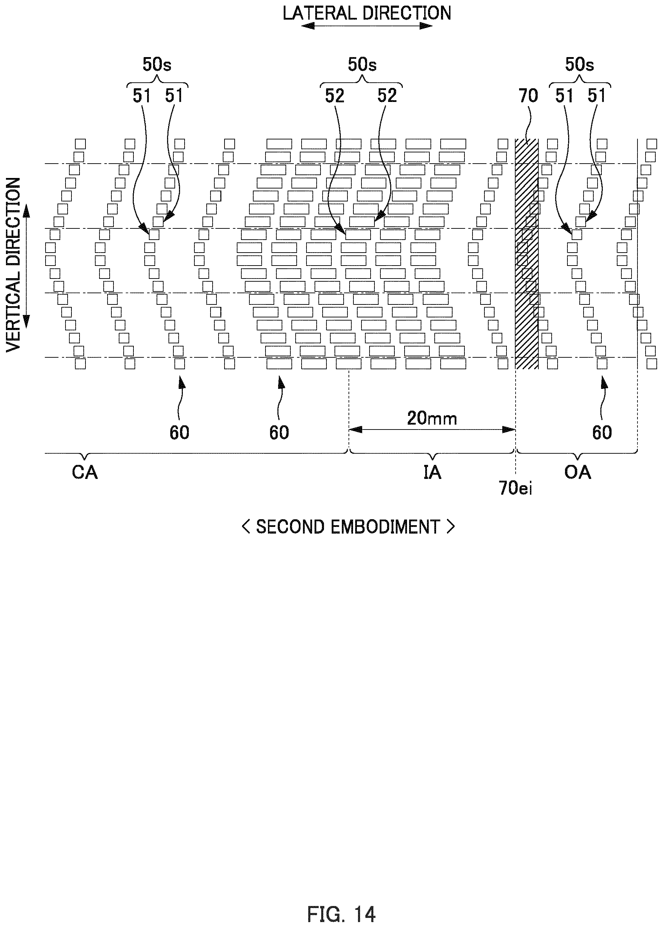

In the front waist portion 31 (41) of the present embodiment, the plurality of welded portions 50, 50, ... are lined up side-by-side in the vertical direction at predetermined intervals, thereby forming a welded portion row 60 extending in the vertical direction. And a plurality of the welded portion rows 60 are provided spacing in the lateral direction. In the example of FIG. 4, the welded portion rows 60 are each arranged straight in the vertical direction, but the welded portion rows 60 may meander with respect to the lateral direction. In other words, the plurality of welded portions 50 that constitute the welded portion row 60 may be located at different positions with respect to the lateral direction. (See the later-described FIG. 14, etc.)

As shown in FIG. 4, the plurality of welded portions 50 are provided at predetermined intervals in the vertical direction and in the lateral direction, and this makes it possible to suppress deterioration of texture of the diaper 1 being put on while maintaining the flexibility of the front waist portion 31 (41). Assuming that a line-shaped welded portion extending lengthwise (continuously) in the vertical direction or in the lateral direction is formed. In a portion where the welded portion is formed, nonwoven fabric becomes stiff. And, it is more likely to suppress deformation of nonwoven fabric in a direction in which the line-shaped welded portion extends. In contrast, the welded portion row 60 in which the small welded portions 50 are dispersed as shown in FIG. 4 makes it possible to provide a flexible sheet member which makes a user (wearer) of diaper 1 less likely to feel curing of nonwoven fabric and makes it less likely to inhibit deformation of nonwoven fabric in the vertical direction and in the lateral direction.

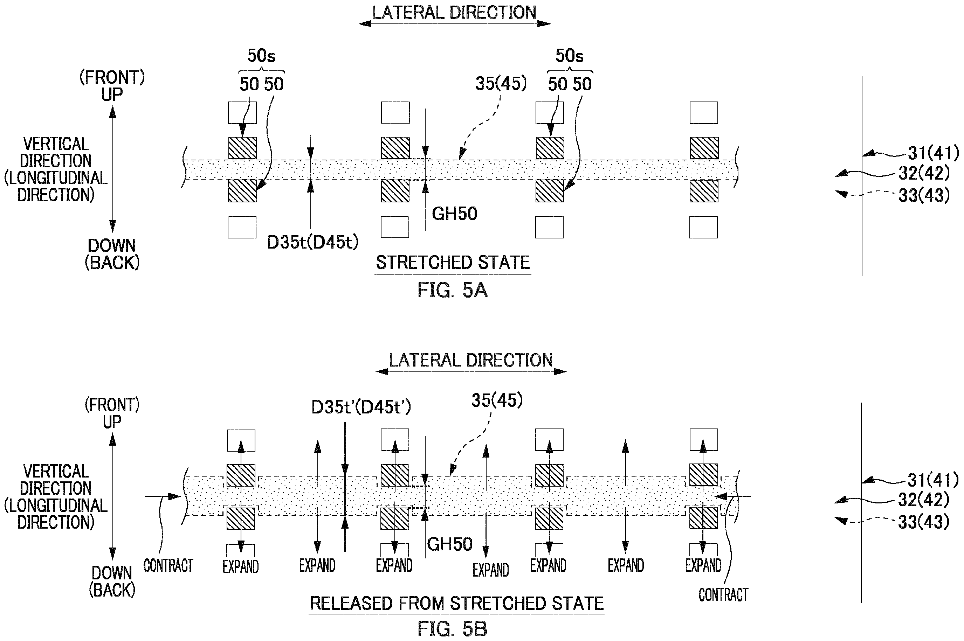

In the front waist portion 31 (41), a waist elastic member 35 (hereinafter simply referred to as elastic member 35 (45)) is sandwiched in the vertical direction between two welded portions 50 and 50 adjacent in the vertical direction, among the plurality of welded portions 50 in the welded portion row 60, and thereby that elastic member 35 is attached to the front waist portion 31 (41). Specifically, a pair of welded portions 50 and 50 respectively located on both sides of the elastic member 35 in the vertical direction serve as welded portion pair 50s, and the elastic member 35 is attached by the welded portion pair 50s. FIG. 5A and 5B are diagrams showing a function to attach an elastic member 35, and are enlarged schematic view of the portion X in FIG. 4.

As shown in FIG. 5A, a pair of welded portions 50 and 50 constituting a welded portion pair 50s are placed side-by-side with a space GH50 in the vertical direction. The size of the space GH50 is set to be equal to or slightly larger than the outer diameter D35t (D45t) of the elastic member 35 (45) which is stretched at a target stretch factor in the lateral direction (GH50 ≥ D35t); the elastic member 35 (45) is an elastic string, for example. In other words, the elastic member 35 in the stretched state is arranged between a welded portion pair 50s in the vertical direction. With such a configuration, in the manufacturing process of the diaper 1 (the front waist portion 31), after arranging each of elastic members 35 between the first sheet 32 and the second sheet 33 with being stretched, when welding the first sheet 32 and the second sheet 33, it is possible to form the welded portion 50 without overlapping the elastic members 35.

When the elastic member 35 (45) is released from the stretched state, the elastic member 35 (45) expands in the vertical direction with contracting in the lateral direction, and its outer diameter D35t' after expansion is larger than the space GH50 of the welded portion pair 50s in the vertical direction (D35t' > GH50), as shown in FIG. 5B. Accordingly, the expansion of the elastic member 35 (45) in the vertical direction is restricted between the welded portion pair 50s, and thus the elastic member 35 (45) is substantially sandwiched between the welded portions 50 and 50 in the vertical direction. Consequently, the elastic member 35 (45) is attached to the front waist portion 31 (41). In addition, in the diaper 1 in the underpants-shaped state in FIG. 1, the elastic members 35 (45) are released from the above-mentioned stretched state.

Incidentally, the foregoing stretch factor is a value R that indicates how many times longer the total length L1 of the elastic member 35 (45) the original total length L0 of the elastic member 35 (45) is (= L1/L0); the total length L0 is a length in a state where in which no force is exerted.

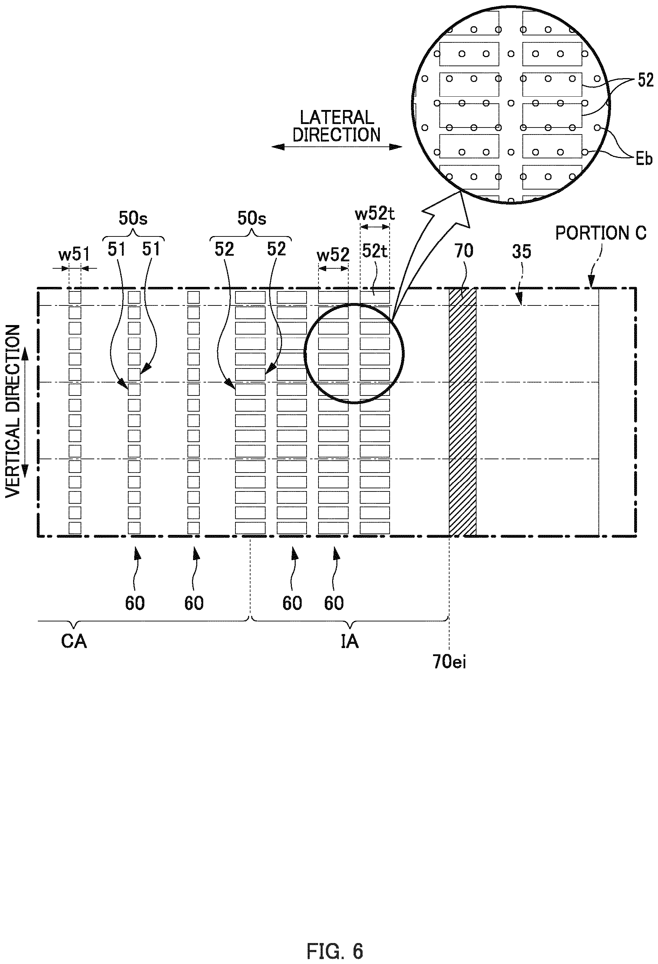

Further, in the diaper 1, a welded portion 50 having a different shape is arranged for each area of the front waist portion 31 (41). FIG. 6 is a diagram illustrating the shape and arrangement of the welded portions 50, and is an enlarged schematic diagram in the portion C in FIG. 4.

As shown in FIG. 6, the welded portions 50 include first welded portions 51 each having a substantially square shape and second welded portions 52 each having a substantially rectangular shape. In the front waist portion 31 (41) that is in the stretched state, the first welded portion 51 and the second welded portion 52 have the same length (height) in the vertical direction, and have different lengths (widths) in the lateral direction. Specifically, the length (width) W52 of the second welded portion 52 in the lateral direction is larger than the length (width) W51 of the first welded portion 51 in the lateral direction (W52 > W51). Accordingly, as described in FIGS. 5A and 5B, when attaching an elastic member 35 (45) by sandwiching by the welded portion pair 50s in the vertical direction, a frictional force when being sandwiched between the wider second welded portions 52 and 52 is larger than a frictional force when being sandwiched between the narrow first welded portions 51 and 51. This makes it easier to prevent the falling off of the elastic member 35 (45) from the welded portion pair 50s (so-called rubber falling off). In addition, in a portion sandwiched by the second welded portions 52 and 52, the amount of stretching/contracting in the lateral direction of the elastic member 35 (45) is smaller than in a portion sandwiched by the first welded portions 51 and 51 (it becomes less likely to contract). The effect according to the difference in shape between the first welded portions 51 and the second welded portions 52 will described explained later.

In the present embodiment, the second welded portion 52, whose width in the lateral direction is wide, is mainly provided in a region adjacent to and inside the side joining portion 70 in the lateral direction. Specifically, in the front waist portion 31 (41), welded portion rows 60 each constituted by the second welded portions 52, 52, ....are provided in a predetermined range inward from the innermost end 70ei of the side joining portion 70 in the lateral direction (see FIGS. 4 and 6). Specifically, when the front waist portion 31 (41) is in the stretched state, the regions that are located above the upper end 10et of the absorbent main body 10 in the vertical direction and within ranges of 20 mm inward from the inner ends 70ei of the side joining portions 70 in the lateral direction are defined as inner end regions IA. At that time, at least one second welded portion 52 is arranged in the inner end region IA.

Similarly, the second welded portion 52 is also provided in a region adjacent to and outside the absorbent core 11c in the lateral direction. Specifically, in the front waist portion 31 (41), welded portion rows 60 each constituted by the second welded portions 52, 52, ....are provided in a predetermined range outward from the outermost end 11ces of the absorbent core 11c in the lateral direction (see FIG. 4). Specifically, when the front waist portion 31 (41) is in the stretched state, the regions that are located below the upper end 10et of the absorbent main body 10 in the vertical direction and within ranges of 20 mm outward from the outer ends 11ces of the absorbent core 11c in the lateral direction are defined as absorbent-body side regions SA. At that time, at least one second welded portion 52 is arranged in the absorbent-body side region SA.

In addition, in the following, the region that is located above the upper end 10et of the absorbent main body in the vertical direction and inside the inner end regions IA in the lateral direction, that is, sandwiched between the pair of inner end regions IA, is defined as a central region CA (see FIG. 4) . Further, the regions that are located above the upper end 10et of the absorbent main body in the vertical direction and outside the inner end regions IA in the lateral direction (outside the inner ends 70ei of the side joining portions 70) are defined as outer end regions OA. Further, the regions that are located below the upper end 10et of the absorbent main body in the vertical direction and are respectively sandwiched between corresponding pairs of the absorbent-body side region SA and the side joining portion 70 in the lateral direction are defined as leg-circumferential regions EA.

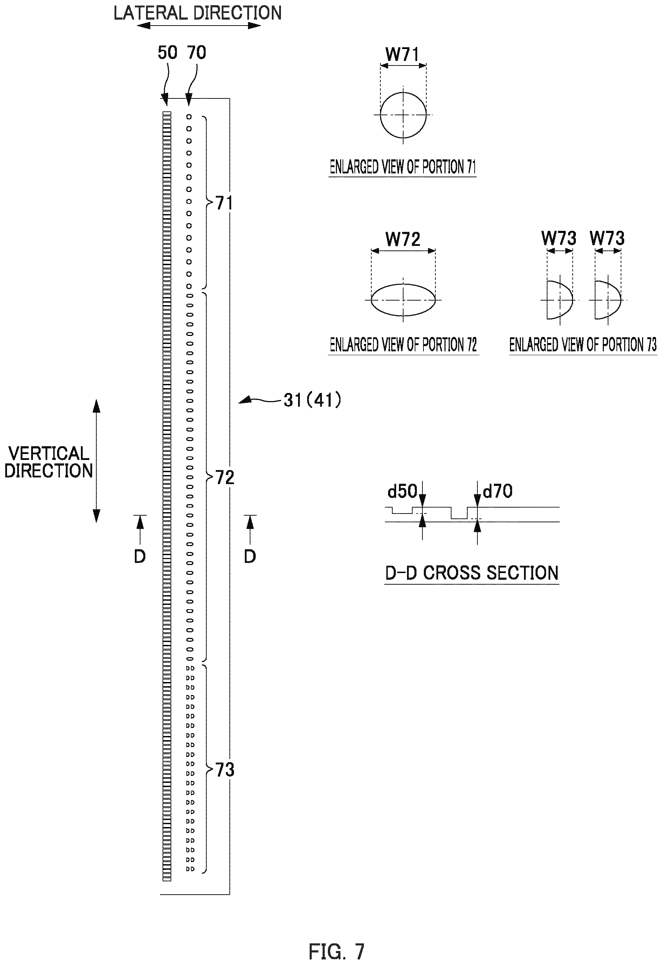

In FIGS. 4 and 6, the side joining portions 70 are illustrated as band-shaped regions extending in the vertical direction, but the actual side joining portions 70 are formed by a plurality of welded portions lined up side-by-side spacing in the vertical direction. FIG. 7 is a diagram illustrating the configuration of the side joining portion 70. In diaper 1, side joining portion 70 has three types of welded portion, first lock welded portions 71, second lock welded portions 72, and third lock welded portions 73, which are formed using known welding means such as ultrasonic welding and thermal welding. The first lock welded portions 71 are each a substantially circular welded portion, and have a size (diameter) of W71 in the lateral direction. The second lock welded portions 72 are each an oval-shaped welded portion whose length in the lateral direction is longer than that in the vertical direction, and have a size (major axis length) of W72 in the lateral direction. The third lock welded portions 73 are each a welded portion in which two substantially semicircular-shaped (semi-elliptical-shaped) welded portions are lined up side-by-side at a predetermined distance in the lateral direction, and each third lock welded portion 73 has a size of W73 in the lateral direction.

The side joining portion 70 is formed by lining up these three types of lock welded portions 71, 72, and 73 from the upper side to the lower side in the vertical direction at a predetermined interval as shown in FIG. 7. Note that the above-mentioned inner end 70ei of the side joining portion 70 is represented by the innermost position of the inner end in the lateral direction for each of the plurality of lock welded portions 71 to 73 that form the side joining portion 70.

### Manufacturing method of diaper 1

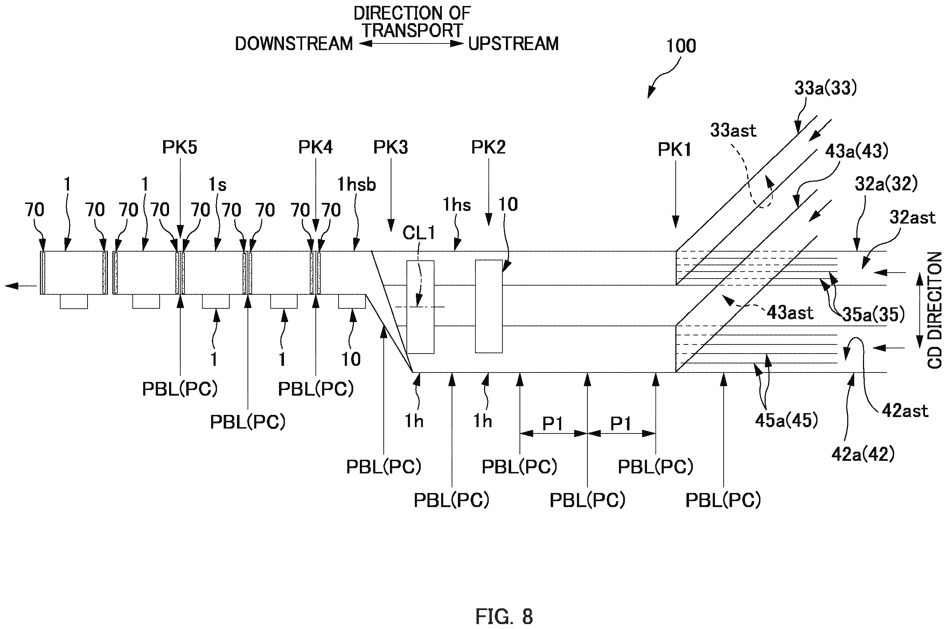

The following describes a method for manufacturing the diaper 1. The diaper 1 is manufactured by a production line 100. FIG. 8 is a schematic plan view showing a partial perspective view of how the diaper 1 is manufactured in the production line 100.

In the production line 100, for example, the two nonwoven fabric sheets 32 and 33 (corresponding to sheet-like member) pertaining to the front waist portion 31 are transported in the form of continuous sheets 32a and 33a (corresponding to sheet-like-member continuous body) that are continuous in the direction of transport, and similarly, the two nonwoven fabric sheets 42 and 43 (corresponding to sheet-like member) pertaining to the back waist portion 41 are transported in the form of continuous sheets 42a and 43a (corresponding to sheet-like-member continuous body), that are continuous in the direction of transport. The sets of two continuous sheets 32a and 33a and two continuous sheets 42a and 43a each pass through the plurality of processing positions PK1 to PK5 that are set in the direction of transport, and are subject to processings that correspond to the processing positions PK1, PK2, and so on.

Note that here, the direction that is orthogonal to both the thickness direction and the direction of transport of the continuous sheets 32a, 33a, 42a, and 43a is defined as "CD direction", and in this example, the continuous sheets 32a, 33a, 42a, 43a, (i.e., two continuous sheets 32a and 33a pertaining to the front waist portion 31 and two continuous sheets 42a and 43a pertaining to the back waist portion 41) are transported side-by-side in the CD direction. However, there is no limitation whatsoever to this.

Further, in this example, the above-described processing positions, namely the first to fifth processing positions PK1 to PK5, are set side-by-side in this order from upstream to downstream in the direction of transport. At the processing positions PK1, PK2, and so on, the processes that are performed on the continuous sheet 32a and 33a pertaining to the front waist portion 31 are substantially the same as those performed on the continuous sheet 42a and 43a pertaining to the back waist portion 41.

For this reason, the front waist portion 31 and the back waist portion 41 will not be distinguished from each other when the same content applies hereinafter. For example, in the following description, the term "waist portion 31 (41)" will simply be used, or the term "two continuous sheets 32a and 33a (42a and 43a) " will simply be used. Note that, in such case, in the term indicating the members, such as "the continuous sheets 32a and 33a (42a and 43a)", "the elastic strings 35 (45)", and "the elastic-string continuous bodies 35a (45a)", the reference signs that immediately follow the terms are the reference signs pertaining to the members associated with the front waist portion 31, and the subsequent reference signs in parentheses are the reference signs of the members associated with the back waist portion 41.

As shown in FIG. 8, the two continuous sheets 32a and 33a (42a and 43a) pertaining to the waist portion 31 (41) are transported in a so-called lateral mode. Specifically, the two continuous sheets 32a and 33a (42a and 43a) are transported such that the direction corresponding to the lateral direction of the diaper 1 conforms to the direction of transport. For this reason, in the two continuous sheet 32a and 33a (42a and 43a), the boundary positions PBL between two diapers 1 that are adjacent in the lateral direction are virtually set at a product pitch P1 in the direction of transport. Also, at the fifth processing position PK5 located at the end of the production line, the boundary position PBL is a cutting target position PC at which the two continuous sheets 32a and 33a (42a and 43a) are cut to produce a single-cut diaper 1.

Note that the two continuous sheets 32a and 33a (42a and 43a) pertaining to the waist portion 31 (41) are transported by an appropriate transporting devices (not shown) such as a belt conveyor or transport rollers. Accordingly, unless otherwise specified, It is assumed that the two continuous sheets 32a and 33a (42a and 43a) are transported in the direction of transport by such transporting devices. Examples of the belt conveyor includes a normal belt conveyor that has an endless belt that is driven to rotate as a transporting surface, and a suction belt conveyor that has a function for suction to the outer circumferential surface of an endless belt.

When manufacturing the diaper 1, as shown in FIG. 8, first, the two continuous sheets 32a and 33a (42a, 43a) pertaining to the waist portion 31 (41) pass the first processing position PK1. While passing, the two continuous sheets 32a and 33a (42a and 43a) are overlaid on each other in the thickness direction. At this time, the elastic-string continuous bodies 35a, 35a, ... (45a, 45a, ...), which are elastic-member continuous bodies and are continuous in the direction of transport, are inserted and arranged side-by-side in the direction of transport between the two mutually-facing surfaces 32ast and 33ast (42ast and 43ast) of the two continuous sheets 32a and 33a (42a and 43a), in a state of being stretched to the above-mentioned target stretch factor in the CD direction (corresponding to an arranging step).

Also, at the same time as this overlaying or immediately thereafter, the above-mentioned welded portions 50, 50,.... are formed as joining portions between the two continuous sheets 32a and 33a (42a and 43a), and thus a pair of facing surfaces 32ast and 33ast (42ast and 43ast) of the two continuous sheets 32a and 33a (42a and 43a) are joined (corresponding to a joining-portion forming step).

Here, in the production line 100, the lateral direction of the diaper 1 conforms to the direction of transport, and the vertical direction of the diaper 1 conforms to the CD direction. For this reason, the welded portions 50 are formed in pairs, on two sides in the CD direction of the elastic-string continuous bodies 35a (45a) . Specifically, two welded portions 50 and 50 that are side-by-side on respective sides in the CD direction of each continuous body 35a (45a) form a welded portion pair 50s. These welded portion pairs 50s are formed side-by-side in the direction of transport spacing between the welded portion pairs 50s that are adjacent in the direction of transport (see FIG. 4, etc.).

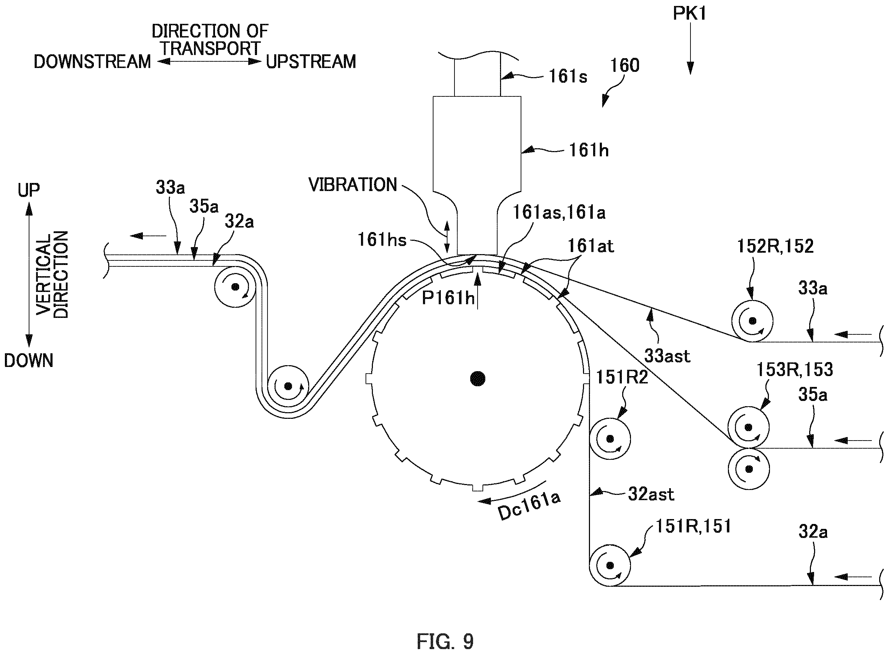

Such welded portions 50 can be formed using a heat sealing device or an ultrasonic welding device 160 (joining-portion formation device), for example (see FIG. 9). Note that a heat sealing device (not shown) has a pair of rolls that are heated while rotating in the direction of transport, for example. One of the rolls is a heat embossing roll that has protrusion portions corresponding to the welded portions 50 on the outer circumferential surface, and the other roll is an anvil roll that receives the protrusion portions with a smooth outer circumferential surface. Further, details of the ultrasonic welding device 160 will be described later.

Next, as shown in FIG. 8, the two continuous sheets 32a and 33a pertaining to the front waist portion 31 and the two continuous sheets 42a and 43a pertaining to the back waist portion 41 all pass the second processing position PK2. At this time, the single-cut absorbent main body 10 produced in a separate process (not shown) is fixed in a state of spanning between the two continuous sheets 32a and 33a pertaining to the front waist portion 31 and the two continuous sheets 42a and 43a pertaining to the back waist portion 41, thus forming the substantially ladder-shaped diaper continuous body 1hs in which substantially H-shaped unfolded diapers 1h, 1h, ... are continuous with each other. The absorbent main body 10 can be fixed using a rotating drum device (not shown), for example. The rotating drum device has a rotating drum that rotates in the direction of transport, and the rotating drum has a plurality of holding portions that detachably hold the absorbent main body 10 to the outer circumferential surface, for example.

Next, the approximately ladder-shaped diaper continuous body 1hs passes the third processing position PK3. At this time, the absorbent main body 10 is folded one time at a predetermined position CL1 of the absorbent main body 10 in the CD direction, and therefore two continuous sheets 32a and 33a pertaining to the front waist portion 31 and two continuous sheets 42a and 43a pertaining to the back waist portion 41 are overlaid on each other in the thickness direction. The folding can be performed using a fold guiding device (not shown), for example. The fold guiding device has a guide plate and a guide roller that are arranged at a predetermined positions in the direction of transport, for example. These guide plates and guide rollers guide the approximately ladder-shaped diaper continuous body 1hs passing at the arrangement position so as to be folded one time.

Next, the diaper continuous body 1hsb in the folded state passes the fourth processing position PK4. At that time, concerning the two continuous sheets 32a and 33a pertaining to the front waist portion 31 and concerning the two continuous sheets 42a and 43a pertaining to back waist portion 41, the two continuous sheets 32a and 33a and 42a and 43a which are overlaid in the thickness direction are welded at positions on two sides of the cutting target position PC in the direction of transport so as to form a pair of side joining portions 70, thereby fixing the diaper continuous body 1hsb in the folded state. This consequently produces an underpants-shaped diaper continuous body 1s in which a plurality of underpants-shaped diapers 1, 1, ... are connected in the lateral direction.

As described in FIG. 7, the side joining portions 70 have a plurality of welded portions 71, 72, 73, ... that are lined up side-by-side in the CD direction (vertical direction). The welded portions 71, 72, and 73 weld together the continuous sheet 33a of the front waist portion 31 and the continuous sheet 43a of the back waist portion 41, and also weld together the pair of facing surfaces 32ast and 33as of the continuous sheets 32a and 33a of the front waist portion 31, and also weld together a pair of facing surfaces 42ast and 43ast of the continuous sheets 42a and 43a of the back waist portion 41.

Such side joining portions 70 can be formed using a heat sealing device (not shown), for example. The heat sealing device (not shown) has a pair of rolls that are heated while rotating in the direction of transport, for example. One of the rolls is a heat embossing roll that has protrusion portions corresponding to the shapes of the welded portions 71, 72, and 73 of the side joining portions 70 on the outer circumferential surface, and the other roll is an anvil roll that receives the protrusion portions with a smooth outer circumferential surface. Note that, depending on the case, the above-mentioned side joining portions 70 may be formed using a welding device having substantially the same configuration as a later-described ultrasonic welding device 160 in FIG. 9.

Next, as shown in FIG. 8, the underpants-shaped diaper continuous body 1s passes the fifth processing position PK5. At this time, the continuous body 1s is cut at the cutting target position PC that is located between a pair of side joining portions 70, thus obtaining the diaper 1.

Note that, at the time of this cutting, the two continuous sheets 32a and 33a (42a and 43a) pertaining to the front waist portion 31 and the back waist portion 41 and the elastic-string continuous bodies 35a, 35a... (45a, 45a...) are cut at the above-mentioned cutting target position. Due to the corresponding relaxation of the stretched state of the elastic strings 35 (45), the elastic strings 35 (45) are sandwiched and pressed by the pair of welded portions 50 in the welded portion pairs 50s, and thus attached to the waist portions 31 and 41, and this is the same as in described in FIG. 4.

The cutting can be performed using a cutter device (not shown), for example. A cutter device has a pair of rolls that rotates in the direction of transport, for example. One of the rolls is a cutter roll that has a cutter blade provided on the outer circumferential surface, and the other roll is an anvil roll having an outer circumferential surface that receives the cutter blade.

Note that, as described above, the processing at the first processing position PK1 is approximately the same for the members 32a, 33a, 35a pertaining to the front waist portion 31 and for the members 42a, 43a, 45a pertaining to the back waist portion 41. Further, in the following description, of the continuous sheets 32a and 33a of the front waist portion 31, one continuous sheet 32a and the other continuous sheet 33a are also referred to as a "first continuous sheet 32a" and a "second continuous sheet 33a", respectively.

FIG. 9 is a diagram illustrating the processing performed at the first machining position PK1. That is, it is a schematic side view of the ultrasonic welding device 160, which is the main equipment for the processing, as seen in the CD direction.

As shown in FIG. 9, at the first processing position PK1, the followings are arranged at positions located upstream in the direction of transport with respect to the ultrasonic welding device 160: a transport mechanism 151 that transports the first continuous sheet 32a in the direction of transport; a transport mechanism 152 that transports the second continuous sheet 33a in the direction of transport; and a transport mechanism 153 that transports the elastic-string continuous bodies 35a in the direction of transport. Note that, the transport mechanisms 151, 152, and 153 respectively includes: transport rollers 151R, 152R, and 153R that rotate around the rotation axis extending along the CD direction; and servo motors (not shown), which are drive sources that drive and rotate the corresponding transport rollers 151R, 152R, and 153R. As a result, the transport roller 151R drives and rotates along the direction of transport to send the first continuous sheet 32a to the ultrasonic welding device 160, and the transport roller 152R also drives and rotates along the direction of transport to send the second continuous sheet 33a to the ultrasonic welding device 160, and the transport roller 153R also drives and rotates along the direction of transport to send the elastic-string continuous bodies 35a to the ultrasonic welding device 160.

On the other hand, the ultrasonic welding device 160 includes: an anvil roll 161a (corresponding to a roll) that rotates along the direction of transport; and a horn 161h that is arranged at a predetermined position P161h in the rotation direction Dc161a of the anvil roll 161a.

The horn 161h is supported so as to be substantially immovable at the predetermined position P161h by an appropriate support member 161s. Further, the horn 161h has a flat vibrating surface 161hs arranged opposite to the outer circumferential surface 161as of the anvil roll 161a. The surface 161hs vibrates in the direction of expanding or reducing the distance from the outer circumferential surface 161as. The frequency of the vibration is, for example, a predetermined value of 20kHz to 35kHz, and the amplitude is, for example, a predetermined value of 1 micron to 30 microns. Therefore, the vibrating surface 161hs vibrates ultrasonically, thereby ultrasonically welding both sheets 32a and 33a that are passing between the vibrating surface 161hs and the outer circumferential surface 161as. That is, the above-mentioned welded portions 50 are formed on both sheets 32a and 33a. Incidentally, such vibrations are generated by inputting an electric signal of the above frequency to piezo elements of a converter (not shown) that is connected to the horn 161h.

The anvil roll 161a is rotatably supported around a rotation axis extending along the CD direction, by an appropriate support member (not shown) such as a bearing. The roll 161a is driven and rotated by being given a driving force from a servo motor (not shown), which serves as a driving source. In addition, there are wound around the roll 161a on its outer circumferential surface 161as with almost no relative slippage, the first continuous sheet 32a sent from the above-mentioned transport roller 151R, the second continuous sheet 33a sent from the above-mentioned transport roller 152R, and the elastic-string continuous bodies 35a sent from the above-mentioned transport roller 153R. Therefore, by driving and rotating the anvil roll 161a, the first continuous sheet 32a, the second continuous sheet 33a, and the elastic-string continuous bodies 35a are transported along the outer circumferential surface 161as of the roll 161a at the same transport speed value as the circumferential speed value of the anvil roll 161a. In other words, both continuous sheets 32a and 33a and the elastic-string continuous bodies 35a are transported in the transport path that curves along the outer circumferential surface 161as.

Here, the circumferential speed value (mpm) of the above-mentioned transport roller 151R of the first continuous sheet 32a and the circumferential speed value (mpm) of the above-mentioned transport roller 152R of the second continuous sheet 33a are approximately the same as the circumferential speed value (mpm) of the anvil roll 161a. Therefore, the first continuous sheet 32a and the second continuous sheet 33a are wrapped around the anvil roll 161a in a state where these sheets are not stretched but tightened to the extent that they do not loosen. On the other hand, the circumferential speed value (mpm) of the transport roller 153R of the elastic-string continuous bodies 35a is approximately 1/ stretch factor times the circumferential speed value (mpm) of the anvil roll 161a. Therefore, while passing between the transport roller 153R and the anvil roll 161a, the elastic-string continuous bodies 35a are stretched to the above-described stretch factor and wraps around the anvil roll 161a in the stretched state.

Further, in this example, regarding the order of wrapping around the anvil roll 161a, first, the first continuous sheet 32a is wrapped, and next the elastic-string continuous bodies 35a are wrapped, and finally the second continuous sheet 33a is wrapped. As a result, these are brought into a state where the elastic-string continuous bodies 35a are interposed between the first continuous sheet 32a and the second continuous sheet 33a, on the outer circumferential surface 61as of the anvil roll 161a.

Furthermore, as shown in FIG. 9, on the outer circumferential surface 161as of the anvil roll 161a, a plurality of protrusion portions 161at, 161at, ... are formed in a protruding manner, corresponding to the aforementioned welded portions 50. When the first and second continuous sheets 32a and 33a between which the elastic-string continuous bodies 35a are interposed pass the arrangement position P161h of the horn 161h because of the rotation of the anvil roll 161a, ultrasonic wave vibration energy is generated from the vibration surface 161hs of the horn 161h so as to be applied to both sheets 32a and 33a. Therefore, the pair of facing surfaces 32ast and 33ast of the sheets 32a and 33a partially generate heat and melt at the positions corresponding to the protrusion portions 161at, and as a result, the plurality of welded portions 50 as mentioned above make the pair of facing surfaces 32ast and 33ast be joined together in a continuously dispersed joining pattern. Then, first continuous sheet 32a and second continuous sheet 33a joined by the welded portions 50 are transported to the aforementioned second processing position PK2 located downstream in the direction of transport, in a state where the elastic-string continuous bodies 35a are interposed therebetween.

Also, it is recommended that the circumference (length in the circumferential direction) of the outer circumferential surface 161as of the anvil roll 161a is made to be the length of one piece or multiple pieces (multiple of one piece) of the underpants-shaped diaper 1 in the direction of transport. For example, n patterns (n=1, 2, 3...) of the joining portion 50 shown in FIG. 4 are formed along the rotation direction of the outer circumferential surface 161as of the anvil roll 161a. And, in the direction of transport (lateral direction of the diaper 1), the welded portions 50 are formed synchronously so that the first welded portions 51 and the second welded portions 52 are formed without shifting their positions. In this way, each of the welded portions 50 (welded portion row 60) can be arranged without timing deviation. Furthermore, if the timing is deviated, the fact is easy to judge visually because the second welded portion 52 overlaps with the side joining portion 70, for example. This makes it possible to accurately judge the quality of the product.

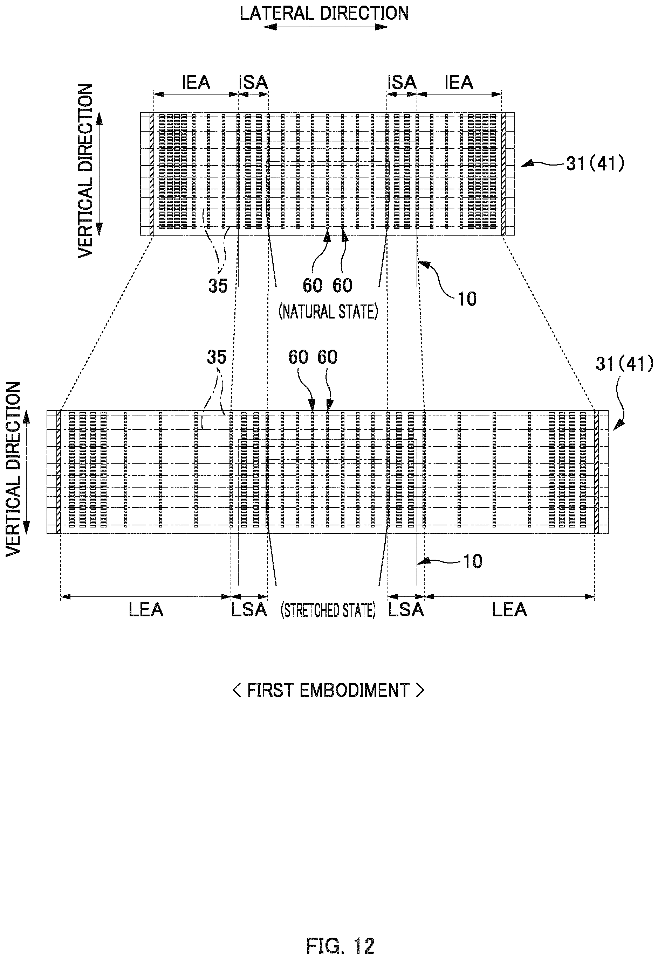

On the other hand, the circumference (length in the circumferential direction) of the outer circumferential surface 161as of the anvil roll 161a may be made to be equal to or shorter than the length of one piece of the underpants-shaped diaper 1 in the direction of transport. For example, a plurality of sets of patterns forming the second welded portions 52 arranged in the inner end region IA are provided in one rotation of the anvil roll 161a. Then, the pattern forming the second welded portions 52 is always arranged in the inner end region IA, and one or more sets of similar patterns are arranged in the central region CA, so that the anvil roll 161a rotates multiple times to form the welded portions 50 (welded portion rows 60) for one piece of the diaper 1. In this way, the roll diameter of the anvil roll 161a can be reduced. Furthermore, since the second welded portions 52 can be formed in the central region CA in the same pattern as the inner end region IA, it becomes possible to suppress the stretching/contracting of the elastic members 35 (45) due to the absorbent-body side region SA and the like. The effect of suppressing the stretching/contracting in the absorbent-body side region SA will be explained with reference to FIG. 12, which will be described later.

### Stretchability of waist portion 20

The waist portion 20 (front waist portion 31 and back waist portion 41) of diaper 1 formed in this way has a different stretchability from conventional diapers because of adjusting the shape and arrangement of the welded portions 50, which have waist elastic members 35 (45) attached to the waist portion 20. The fact that the stretchability is different makes it possible to improve the ease of putting on and taking off the diaper compared to conventional diapers.

For example, at the time of using the underpants-shaped disposable diaper, when removing the diaper from the wearer's body, it is common to conduct an operation to spread open the waist opening BH and the leg openings LH by tearing the side joining portions (70), which join the front waist portion (31) and the back waist portion (41). At this time, with the conventional underpants-shaped diaper, the side joining portions (70) may be less likely to tear due to stretchability of the front waist portion (31) and the back waist portion (41) in the lateral direction. That is, in order to tear the side joining portions 70, when holding the front waist portion (31) and back waist portion (41) in the vicinity of the side joining portion 70 and pulling it to the opposite sides in the front-back direction, there are cases where the front waist portion (31) and the back waist portion (41) stretch in the lateral direction, which is made it less likely for the force to act on the side joining portion 70. In addition, a force has been consumed to stretch the front waist portion (31) and the back waist portion (41), making it difficult to efficiently perform the action of tearing the side joining portion (70).

In contrast, in the diaper 1, by weakening lateral stretchability of the front waist portion 31 and the back waist portion 41 in regions near the side joining portions 70, a force becomes more likely to act on the side joining portions 70, making it possible to efficiently tear the side joining portions 70 with a small force.

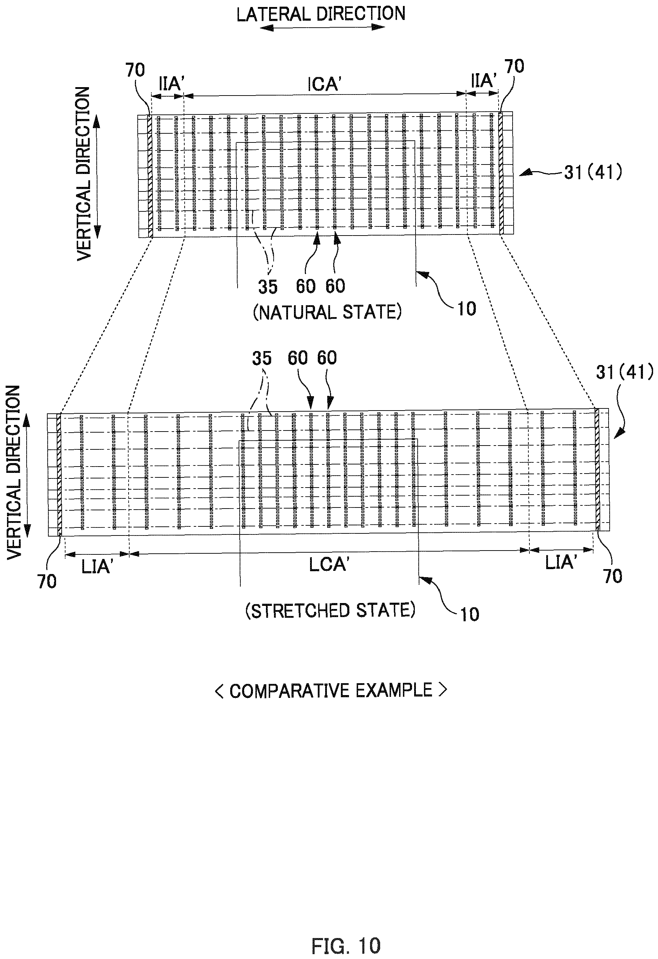

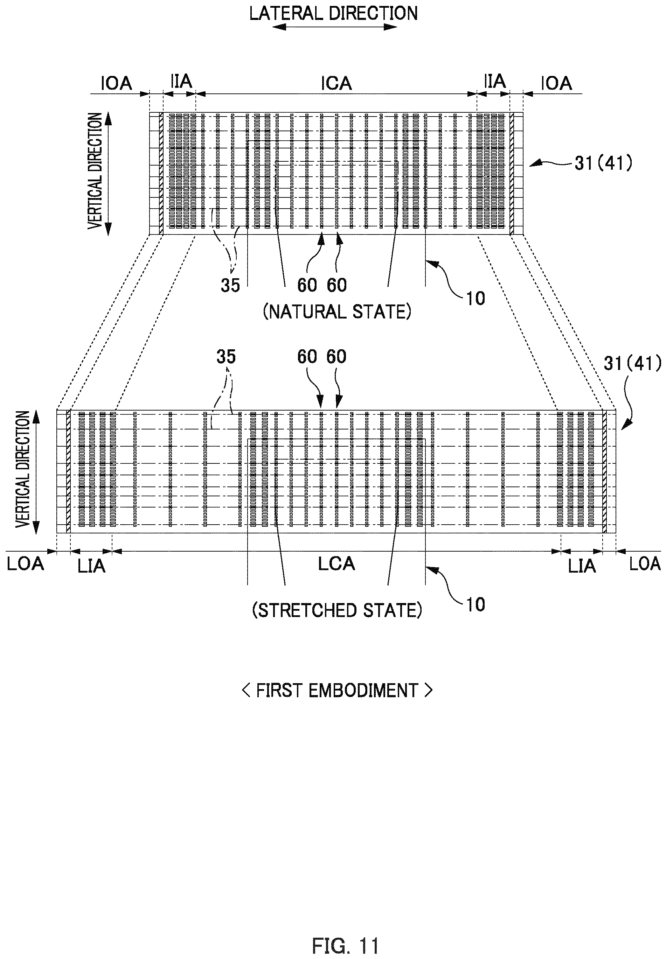

FIG. 10 is a diagram illustrating the stretchability of the front portion 31 (41) in conventional underpants-shaped diapers as a comparative example. FIG. 11 is a diagram illustrating the stretchability of the front waist portion 31 (41) in the diaper 1 of the first embodiment. The basic configuration of the front waist portion 31 (41) of the comparative example shown in FIG. 10 is substantially the same as the front waist portion 31 (41) of the diaper 1, and the shape and arrangement of the welded portions 50 (welded portion rows 60) are different from those of the diaper 1. That is, in the diaper 1, the welded portion rows 60 are each constituted by two types of the welded portions 51 and 52 as described in FIG. 4 and the like, whereas in the comparative example, the welded portion rows 60 are constituted by arranging one type of welded portion (one corresponding to the first welded portion 51 of the diaper 1) at regular intervals in the vertical direction and the lateral direction. In addition, the regions (central region CA', inner end region IA', outer end region OA') that are substantially the same as the regions (central region CA, inner end region IA, outer end region OA, etc.) set for the front waist portion 31 (41) of the diaper 1 described in FIG. 4 are set for the front waist portion 31 (41) of the comparative example.

Furthermore, in FIGS. 10 and 11, the diagrams shown at the top represent the plan views of the front waist portion 31 (41) in a natural state, and the diagrams shown at the bottom represent the plan view of the front waist portion 31 (41) in the stretched state (41). The "natural state" is the state when the diaper 1 and the front waist portion 31 (41) are left on its own for a predetermined period of time. For example, the front waist portion 31 and the back waist portion 41 of the diaper 1 are pulled outward on both sides in the lateral direction to put the waist portions 31 and 41 in the "stretched state", the stretched state is maintained for 15 seconds, and then the diaper 1 is released and placed on a flat surface such as a desk. The state after 5 minutes of being laid flat on the flat surface is defined as the natural state.

In the front waist portion 31 (41) of the comparative example shown in FIG. 10, the lateral length (width) of the central region CA' in the natural state is a length lCA', and the lateral length (width) of the inner end region IA' is a length lIA'. When the front waist portion 31 (41) changes from the natural state to the stretched state, the lateral length (width) of the central region CA' is a length LCA', and the lateral length (width) of the inner end region IA' is a length LIA'.

The lateral lengths (width) of the central region CA' and inner end region IA' are each the average value of the lateral length (width) at the upper end position in the vertical direction and the lateral length (width) at the lower end position in the vertical direction. When the front waist portion 31 (41) stretches and contracts, there is a risk that the closer portions their vertical distances to the absorbent main body 10, which has higher stiffness, the less likely the portions are made to stretch and contract, because of the influence of the absorbent main body 10. That is, there is a possibility of a difference in the lateral length (width) between the upper end position and the lower end position in the vertical direction. Therefore, by taking the average value of the length (width) at the upper end position in the vertical direction and the length (width) at the lower end position in the vertical direction, it is possible to make as small as possible the error that may occur depending on the measurement position. In this specification, the definition of the lateral length (width) of each region (IA, CA, OA, SA, EA, etc.) is the same hereinafter.

Here, the ratio of change in the length of the central region CA' in the lateral direction when the front waist portion 31 (41) changes from the natural state to the stretched state (hereinafter also referred to as the "dimensional change ratio") is represented by a value calculated as follows: the amount of stretching (contracting) of the central region CA' (LCA' - lCA') is divided by the length LCA' at the time of stretching. That is, the dimensional change ratio of the central region CA' is indicated by (LCA' - lCA')/LCA'.

Similarly, the ratio of change in the length of the inner end region IA' in the lateral direction when the front waist portion 31 (41) changes from the natural state to the stretched state (dimensional change ratio) is indicated by (LIA' - lIA')/LIA'.

Then, in the front waist portion 31 (41) of the comparative example, the dimensional change ratio of the central region CA' ((LCA' - lCA')/LCA') and the dimensional change ratio the inner end region ((LIA' - lIA')/LIA') are substantially equal. This is because the welded portions 50 (welded portion rows 60) by which the waist elastic members 35 are attached is evenly arranged, so that stretchability exhibits evenly throughout the front waist portion 31 (41). Therefore, when attempting to tear the side joining portion 70 during putting on or taking off the diaper as described above, the front waist portion 31 (41) is likely to extend in the lateral direction as a whole, making it difficult to efficiently apply a force to the side joining portion 70. Furthermore, due to the influence of the stiffness of the absorbent main body 10, there is a possibility that the dimensional change ratio of the central region CA' is slightly smaller than the dimensional change ratio of the inner end region IA', but in the comparative example, the front waist portion 31 (41) is likely to stretch in the lateral direction as a whole.

Next, in the front waist portion 31 (41) of the present embodiment shown in FIG. 11, the lateral length (width) of the central region CA in the natural state is a length lCA, and the lateral length (width) of the inner end region IA is a length lIA. Further, when the front waist portion 31 (41) changes from the natural state to the stretched state, the lateral length (width) of the central region CA is a length LCA, and the lateral length (width) of the inner end region IA is a length LIA. Then, dimensional change ratio of the central region CA is indicated by (LCA - lCA)/LCA, and the dimensional change ratio of the inner end region IA is indicated by (LIA - lIA)/LIA.

In the front waist portion 31 (41) of the diaper 1 of the first embodiment, unlike the case of the comparative example, the dimensional change ratio of the inner end region IA ((LIA - lIA)/LIA) is smaller than the dimensional change ratio of the central region CA ((LCA - lCA)/LCA). That is, concerning the lateral dimensional change ratio of an elastic member 35 (45) attached by welded portions 50 (welded portion pair 50s) (ratio of change in the lateral length of the elastic member) from the natural state to the stretched state, the lateral dimensional change ratio in the inner end region IA is smaller than the lateral dimensional change ratio in the central region CA.

With this configuration, when attempting to tear the side joining portion 70 during the putting on and taking off of the diaper 1, the front waist portion 31 (41) is less likely to stretch/contract in the lateral direction in the inner end region IA, which is located near the side joining portion 70, compared to in the central region CA. Therefore, when holding the front waist portion (31) and the back waist portion (41) and pulling them respectively to the opposite sides in the front-back direction, the force is likely to efficiently act on the side joining portion 70, making it possible to easily tear the side joining portion 70. This can make it easier to remove the diaper 1 from the wearer's body by tearing the side joining portions 70.

In the diaper 1 of the present embodiment, the second welded portions 52, each of which has a wider range than the first welded portion 51, are provided in the inner end region IA of the front waist portion 31 (41). In other words, the width W52 of at least one of the second welded portions 52 located in the inner end region IA is longer than the minimum value of the width W51 of the first welded portions 51 located in the central region CA. (see FIG. 6). With such a configuration, a wide lateral range of each waist elastic member 35 which is attached by the second welded portions 52 in the inner end region IA is sandwiched with a pair of second welded portions 52 and 52 (welded portion pair 50s). This makes the waist elastic members 35 less likely to stretch/contract in the lateral direction compared to in the central region CA, and prevents the inner end regions IA of the front waist portion 31 (41) from stretching excessively. This makes a force likely to act on the side joining portion 70, making it possible to easily tear the side joining portion 70 when removing the put-on diaper 1 from the body.

However, if the stretchability in the inner end regions IA can be weakened compared to in the central region CA, it is possible to vary the shape and arrangement of the welded portions 50 as appropriate. For example, the welded portions may be arranged such that the widths of the welded portions 50 per unit length in the inner end region IA is larger than the widths of the welded portions 50 per unit length in the central region CA. In other words, in the case where the value obtained by dividing the sum of the lateral widths of the welded portions 50 located in the inner end region IA by the lateral width of the inner end region IA is greater than the value obtained by dividing the sum of the lateral widths of the welded portions 50 located in the central region CA by the lateral width of the central region CA, it is possible to make the stretchability in the inner end regions IA smaller than in the central region CA. Even with such a configuration, it makes a force likely to efficiently act on the side joining portion 70, making it possible to easily tear the side joining portion 70.

In addition, in FIG. 6, within the inner end regions IA of the front waist portion 31 (41), the welded portion 50 which is located in the lateral direction nearest to the inner end 70ei of the side joining portion 70 is defined as a second welded portion 52t. At this time, it is preferable that the lateral width W52t of the second welded portion 52t is larger than the lateral width W50 of at least one of other welded portions 50 located inside the second welded portion 52t in the lateral direction. That is, it is preferable that the second welded portion 52t is wider than at least one of the other second welded portions 52 and the first welded portions 51. In this case, it is possible to reduce lateral stretching/contracting in a region within the inner end region IA that is nearest to the side joining portion 70. This makes a force likely to more efficiently act on the side joining portion 70, making it possible to more easily tear the side joining portion 70. Furthermore, since the width of the second welded portion 52t that is close to the side joining portion 70 is wide, it increases the lateral frictional force with respect to the waist elastic member 35 in the second welded portion 52t, making it less likely to cause so-called rubber falling off.

In addition, in the front waist portion 31 (41) of the diaper 1, letting the lateral length (width) of the outer end region OA in the natural state be a length lOA, and letting the lateral length (width) of the outer end region OA in the stretched state be a length LOA (FIG. 11), the dimensional change ratio of the outer end region OA is indicated by (LOA - lOA)/LOA. At this time, it is preferable that the dimensional change ratio (LOA - lOA)/LOA of the outer end region OA is smaller than the dimensional change ratio (LIA - lIA)/LIA of the inner end region IA. With such a configuration, it reduces lateral stretching/contracting in the outer end region OA, which includes the side joining portion 70, and this makes the force that will tear the side joining portion 70 more likely to act. Furthermore, the outer end regions OA are less likely to stretch/contract, making it easier to hold those regions with hands. In addition, it makes it more likely to transmit the force that holds the outer end region OA and pulls the front waist portion 31 and the back waist portion 41 respectively to the opposite sides, and makes it easier insert fingers into the inner end regions IA. This makes is possible to easily tear the side joining portion 70.

As described in FIG. 8, in the present embodiment, in the manufacturing process, when cutting an underpants-shaped-diaper continuous body 1s at the cutting target position PC between a pair of side joining portions 70 and 70, the waist elastic members 35 (continuous bodies 35a) are also cut together. At this time, the waist elastic members 35 contract in the lateral direction from the cutting target positions PC toward the side joining portions 70, making it less likely to generate stretchability in the outer end regions OA. Therefore, in the outer end regions OA, the dimensional change ratio between the natural state and the stretched state is an extremely small, and accordingly the outer end regions OA have a configuration that makes it likely to obtain the above-described effects.

Further, in the back waist portion 41 of the diaper 1, the elastic members 45 are provided also in the buttocks cover 41c, which is located below the lower end of the side joining portion 70 in the vertical direction (see FIG. 2). The elastic members 45 are each attached to the buttocks cover 41c by being sandwiched in the vertical direction with the welded portion pair 50s similarly to the manner described in FIG. 5. It is preferable that the lateral length (width) of the welded portion 50 (not shown) which is located nearest to and laterally inside the outer edge portion of the buttocks cover 41c (that is, the outline of the leg opening LH) is larger than the lateral length (width) of at least one of other welded portions 50 located inside the foregoing welded portion 50.

When removing the put-on diaper 1 from the wearer's body, an operation of tearing the side joining portion 70 from the lower side to the upper side in the vertical direction may be performed. For example, when a caregiver takes off the diaper 1 from a bedridden cared person, it may be easier to tear the side joining portion 70 from the lower side than to tear from the upper side. In such a case, the lower end portion of the front waist portion 31 and the buttocks cover 41c of back waist portion 41 are held and pulled respectively to opposite directions, but if the buttocks cover 41c stretches excessively at that time, there is a risk that a force is less likely to act on the side joining portion 70. In contrast, the width of welded portions 50 in regions close to the outer edge portions of the buttocks cover 41c is made wider to make the waist elastic members 45 less likely to stretch/contract, and accordingly it makes a force likely to act on the lower end portion of the side joining portion 70, making it possible to easily tear the side joining portion 70 from the lower side.

Further, as described above, spunbond nonwoven fabric can be used as both of the first sheet 32 (42) and the second sheet 33 (43) that constitute the front waist portion 31 (41) of the diaper 1. Generally, on the surface of such a spunbond nonwoven fabric sheet, a plurality of dot-shaped heat fused portions EB for fusing together heat-fusing fibers made of thermoplastic resin, etc. are provided in a dispersed manner (see FIG. 6). In the front waist portion 31 (41) of the diaper 1, at least one of the welded portions 50 (second welded portions 52) located in the inner end region IA has a portion that overlaps with a heat fused portion EB when seen in the thickness direction.

In the portion where the heat fused portion EB and the welded portion 50 overlap, protrusions/recesses in the thickness direction are deeper, making fingers likely to become caught compared to other regions. This makes a user easier to hold the inner end regions IA of the front waist portion 31 (41), making it possible to easily conduct an operation of tearing the side joining portion 70.

Further, as described in FIG. 7, the side joining portions 70 of the front waist portion 31 (41) are formed by a plurality of the lock welded portions 71 to 73. It is preferable that the depth d70 of the lock welded portions 71 to 73 in the thickness direction, which constitute the side joining portion 70, is deeper than the depth d50 of the welded portion 50 in the thickness direction, which is for attaching the elastic member 35 (45) (d70 > d50). Due to the difference in depth between the lock welded portions 71 to 73 and the welded portion 50, when a user holds the inner end region IA and the outer end region OA of the front waist portion 31 (41), the user can feel the difference in touch between the side joining portion 70 and the remaining portion. In other words, by intuitively understanding that the thickness and stiffness in the front waist portion 31 (41) are different, the user can recognize the portion where the side joining portion 70 is formed, without making a mistake. This makes it easier to facilitate an operation of holding the inner end region IA and the outer end region OA and tearing the side joining portion 70.

In addition, it is preferable that the maximum value of the lateral length (width) of the plurality of lock welded portions 71 to 73 is smaller than the maximum value of the lateral length (width) of the welded portions 50 provided in the inner end region IA (second welded portions 52). That is, it is preferable that, among the width W71 of the first lock welded portion 71, the width W72 of the second lock welded portion 72, and the width W73 of the third lock welded portion 73, the largest value (width W72 in FIG. 7) is smaller than the width W52 of the second welded portion 52 (see FIG. 6). With this configuration, compared to the case where the width W52 of the second welded portion 52 is smaller than the maximum value of the widths of the lock welded portions 71 to 73, it is likely to suppress the stretching/contracting of the waist elastic members 35 (45) in the inner end region IA. This makes a force likely to act on the side joining portion 70 when conducting the attachment/detachment operation, making it possible to easily tear the side joining portion 70.

Further, in the front waist portion 31 (41) of the diaper 1, the second welded portion 52 may be provided in a region other than the inner end regions IA. In the present embodiment, as shown in FIG. 4, the second welded portions 52 are also provided in the absorbent-body side regions SA, each of which is a region extending 20 mm outward from the outer end 11ces of the absorbent core 11c. And it is preferable that the lateral dimensional change ratio of the absorbent-body side regions SA is smaller than the lateral dimensional change ratio of the leg-circumferential regions EA, which are adjacent to the absorbent-body side regions SA on both sides.

FIG. 12 is a diagram illustrating the stretchability in the absorbent-body side region SA of the front waist portion 31 (41) . In FIG. 12, the lateral length of the absorbent-body side region SA in the natural state is defined as a length lSA, and the lateral length of the leg-circumferential region EA in the natural state is defined as a length lEA. Further, the lateral length of the absorbent-body side region SA in the stretched state is defined as a length LSA, and the lateral length of the leg-circumferential region EA in the stretched state is defined as a length LEA. At this time, it is preferable that the lateral dimensional change ratio of the absorbent-body side region SA ((LSA - lSA)/LSA) is smaller than lateral dimensional change ratio of the leg-circumferential region EA (LEA - lEA)/LEA.

The absorbent-body side region SA is a region that, when putting on the diaper 1, is held and pulled up with a hand in order to fit the absorbent core 11c to the wearer's crotch. Therefore, if the dimensional change ratio of this absorbent-body side region SA is large and the absorbent-body side region SA is likely to stretch/contract in the lateral direction, the absorbent core 11 is less likely to be pull upward, causing a risk of not obtaining a good fit. In contrast, in the case where the dimensional change ratio of the absorbent-body side region SA is small, when pulling the absorbent-body side region SA upward, the absorbent core 11c is also likely to be pulled upward accordingly, making it possible to increase the fit of the absorbent core 11c in the crotch portion.

Further, as shown in FIG. 1, a plurality of wrinkles extending in the vertical direction are formed on the surface of the front waist portion 31 and the back waist portion 41 of the diaper 1 in the natural state. These wrinkles are generated by the protruding deformation of the first sheet 32 (42) and the second sheet 33 (43), which constitute the front waist portion 31 (41), in the thickness direction when the waist elastic members 35 (45) contracts in the lateral direction. It is preferable that at least some of these wrinkles are continuous between two waist elastic members 35 (45) arranged adjacent to each other in the vertical direction. In other words, among the plurality of the waist elastic members 35 (45) provided in the front waist portion 31 (41), it is preferable that continuous wrinkles are formed between a first waist elastic member 35A (45A) and a second waist elastic member 35B (45B) in the vertical direction (both are not shown); the first waist elastic member 35A (45A) and the second waist elastic member 35B (45B) being respective one of two waist elastic members 35 (45) that are adjacent in the vertical direction.

Due to the formation of continuous wrinkles along vertical direction, when a wearer holds the front waist portion 31 (41) and pulls it in the lateral direction, it makes the fingers likely to become caught in the wrinkles and less likely to slip. This can make it easier to stably perform the operation of tearing the side joining portion 70.

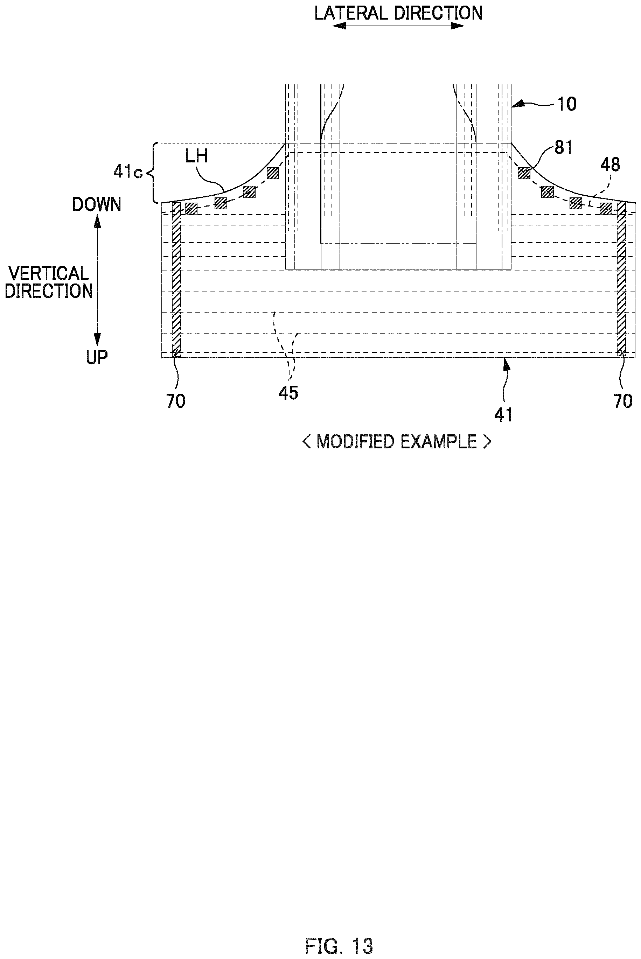

FIG. 13 is a schematic plan view illustrating a modified example of the back waist portion 41. In the modified example shown in FIG. 13, the waist elastic member 45 is not arranged in the buttocks cover 41c of the back waist portion 41, and instead a leg elastic member 48 is arranged which stretches/contracts along the outer edge portion of the buttocks cover 41c (that is, the outlines of the leg openings LH). The leg elastic member 48 is not attached to the buttocks cover 41c by the welded portions 50, but is attached to the buttocks cover 41c by leg-elastic-member adhesive portions 81, which are formed by applying adhesive, such as hot-melt adhesive, to a predetermined region. As shown in FIG. 13, a plurality of the leg-elastic-member adhesive portions 81 are provided along the contours of the leg openings LH, and as a result, the leg elastic member 48 exhibits stretchability along the leg openings LH, making it possible to suppress rolling up of the buttocks cover 41c after fitting to the wearer's buttocks.

Since the leg elastic member 48 is attached by leg-elastic-member adhesive portion 81, it increases the stiffness of the region extending along the outer edge portion of the buttocks cover 41c (the outlines of the leg openings LH). Therefore, at the time of removing the diaper 1 from the body, when performing an operation of tearing the side joining portion 70 from the lower side to the upper side, even if the buttocks cover 41c is pulled strongly, the fabric (nonwoven fabric) is less likely to tear, making it possible to easily tear the side joining portion 70.

Note that the leg-elastic-member adhesive portions 81 are arranged in a curved manner along the leg openings LH. In this curved portion, the leg-elastic-member adhesive portions 81 are attached being more stretched than in the remaining portion. Therefore, the stretching/contracting range of the leg-elastic-member adhesive portions 81 in regions adjacent to and inside the side joining portion 70 in the lateral direction (regions that overlap respectively the inner end regions IA with respect to the lateral direction) is smaller than the stretching/contracting range of the leg-elastic-member adhesive portions 81 in a region adjacent to and inside the foregoing regions (region that overlap the central region CA with respect to the lateral direction). Therefore, by arranging the leg-elastic-member adhesive portions 81 in a curved manner along the leg openings LH, it can make the lower end portion of the side joining portion 70 easier to tear.

### Second embodiment

In the second embodiment, an example will be described in which the arrangement of the welded portions 50 provided in the front waist portion 31 and the back waist portion 41 are different from the first embodiment. FIG. 14 is a diagram illustrating the shape and arrangement of the welded portions 50 of the second embodiment, and corresponds to FIG. 6 of the first embodiment. Note that the basic configuration of the disposable diaper of the second embodiment is substantially the same as the diaper 1 of the first embodiment, and the arrangement of the welded portions 50 is different from that of the diaper 1.

In the second embodiment, the welded portions 50 provided the front waist portion 31 and the back waist portion 41 form welded portion rows 60 that meander in the lateral direction, as shown in FIG. 14. In other words, two welded portions 50 and 50 adjacent in the vertical direction are arranged so that their portions are located being shifted from each other in the lateral direction, and this makes the welded portions form convex parts on both sides in the lateral direction. However, the shape of the welded portion rows 60 is not limited to that shown in FIG. 14, and the arrangement of the welded portions 50 is not limited to the example shown in FIG. 14. For example, the welded portion row 60 may have a portion that is convex only on the one side in the lateral direction.

By changing the arrangement of the welded portion rows 60 in this way, it is possible to adjust wrinkles formed on the surfaces of the front waist portion 31 and the back waist portion 41. For example, by making the welded portion rows 60 meander in the lateral direction as shown in FIG. 14, it makes irregular the shape and size of the wrinkles formed between two welded portion rows 60 and 60 adjacent in the lateral direction, making it possible to make soft the texture of the front waist portion 31 (41) and to make it easier to create a natural texture.

In the second embodiment, as in the first embodiment, the width of the welded portions 50 provided in the inner end regions IA, which are located within the range of 20 mm inward from the inner ends 70ei of the side joining portions 70 in the lateral direction is wider than the width of the welded portions 50 provided in the central region CA, which is located inside the inner end region IA. That is, at least the second welded portion 52, which is wider than the first welded portion 51 provided in the central region CA, is provided in the inner end region IA. Therefore, in the front waist portions 31 (41), the dimensional change ratio of the inner end region IA is smaller than the dimensional change ratio of the central region CA. This makes the inner end regions IA less likely to stretch/contract excessively in the lateral direction, so that when attempting to tear the side joining portion 70, a force is more likely to act on the side joining portion 70. This can make it possible to easily tear the side joining portion 70.

In addition, in the second embodiment, the welded portion rows 60 constituted by the first welded portions 51 are provided in the outer end regions OA, which are respectively adjacent to and outside the inner end regions IA. This increases the stiffness of the outer end regions OA, and, when tearing side joining portion 70, it is possible to make it easier to prevent the front waist portion 31 (41) from being torn. Further, it is possible to easily suppress falling off of rubber of the waist elastic member 35 (45) in the outer end regions OA.

### Other embodiments

Although the embodiments of the present disclosure have been described hereinabove, the above embodiments of the present disclosure are simply to facilitate understanding of the present disclosure and are not in any way to be construed as limiting the present disclosure. The present disclosure may variously be changed or altered without departing from its gist and encompass equivalents thereof. For example, the following modifications are possible.

### REFERENCE SIGNS LIST

1. diaper (absorbent article, underpants-shaped disposable diaper),
10. absorbent main body,
10ea end, 10eb end, 10et upper end,
11. absorbent body, 11c absorbent core, 11ces end,
13. top sheet, 15 back sheet,
17. elastic string, 18 elastic string,
20. waist portion,
31. front waist portion, 31e end,
32. first sheet, 33 second sheet,
35. elastic member,
41. back waist portion, 41c buttocks cover, 41e end,
42. first sheet, 43 second sheet,
45. elastic member, 48 leg elastic member,
50. welded portion, 50s welded portion pair,
51. first welded portion, 52 second welded portion,
60. welded portion row,
70. side joining portion, 70ei inner end,
71. first lock welded portion, 72 second lock welded portion, 73 third lock welded portion,
81. leg-elastic-member adhesive portion,
100. production line,
151. transport mechanism, 151R transport roller,
152. transport mechanism, 152R transport roller,
153. transport mechanism, 153R transport roller,
160. ultrasonic welding device,
161a anvil roll, 161at protrusion portion, 161h horn,
BH waist opening, LH leg opening,
CL1 center position,
CA central region, IA inner end region, OA outer end region,
SA absorbent-body side region, EA leg-circumferential region,
EB heat fused portion

## Claims

1. An underpants-shaped absorbent article having a vertical direction and a lateral direction that intersect with each other, the underpants-shaped absorbent article comprising:
an absorbent main body; and
a waist portion in which a front waist portion and a back waist portion are joined to each other by a pair of side joining portions in two ends in the lateral direction;
the waist portion having a stretchability in the lateral direction, in at least a portion located between the pair of side joining portions,
the waist portion that is in a stretched state having:
inner end regions that are located above an upper end of the absorbent main body in the vertical direction and within a range of 20 mm inward from an inner end of the side joining portion in the lateral direction; and
a central region that is located above the upper end of the absorbent main body in the vertical direction and is sandwiched between a pair of the inner end regions in the lateral direction,
concerning a difference between a lateral length of the inner end region in the stretched state and a lateral length of the inner end region in a natural state,
concerning a difference between a lateral length of the central region in the stretched state and a lateral length of the central region in the natural state,
a value obtained by dividing the difference of the inner end region by the lateral length of the inner end region in the stretched state
being smaller than
a value obtained by dividing the difference of the central region by the lateral length of the central region in the stretched state.

2. The underpants-shaped absorbent article according to claim 1, wherein
the waist portion has an outer end region that is located outside the inner end of the side joining portion in the lateral direction, and
concerning a difference between a lateral length of the outer end region in the stretched state and a lateral length of the outer end region in the natural state,
concerning the difference between a lateral length of the inner end region in the stretched state and the lateral length of the inner end region in the natural state,
a value obtained by dividing the difference of the outer end region by the lateral length of the outer end region in the stretched state
is smaller than
a value obtained by dividing the difference of the inner end region by the lateral length of the inner end region in the stretched state.

3. The underpants-shaped absorbent article according to claim 1 or 2, wherein
the waist portion has a skin-side sheet and a non-skin-side sheet that are stacked in a thickness direction,
a plurality of elastic members are arranged spacing in the vertical direction between the skin-side sheet and the non-skin-side sheet that are joined by a plurality of welded portions,
the plurality of elastic members being capable of stretching and contracting in the lateral direction,
each of the elastic members is attached, in a state of contracting in the lateral direction, to the skin-side sheet and the non-skin-side sheet by being sandwiched between two vertically adjacent ones of the plurality of welded portions,
concerning a difference between a lateral length of an elastic member in the stretched state and a lateral length of the same elastic member in the natural state,
a value obtained by dividing the difference of the elastic member that is attached by the welded portion located in the inner end region by the lateral length of the same elastic member in the stretched state
is smaller than
a value obtained by dividing the difference of the elastic member that is attached by the welded portion located in the central region by the lateral length of the same elastic member in the stretched state.

4. The underpants-shaped absorbent article according to claim 3, wherein
in the stretched state,
a lateral width of at least one of the welded portion located in the inner end region is greater than a minimum value of a lateral width of the welded portion located in the central region.

5. The underpants-shaped absorbent article according to claim 3 or 4, wherein
in the stretched state,
a value obtained by dividing a sum of a lateral width of the welded portion located in the inner end region by a lateral width of the inner end region
is greater than
a value obtained by dividing a sum of a lateral width of the welded portion located in the central region by a lateral width of the central region.

6. The underpants-shaped absorbent article according to any one of claims 3 to 5, wherein
a lateral length of the welded portion that is located in the lateral direction nearest to an inner end of the side joining portion
is longer than
a lateral length of at least one of other welded portion that is located inside the welded portion that is located in the lateral direction nearest to an inner end of the side joining portion.

7. The underpants-shaped absorbent article according to any one of claims 3 to 6, wherein
the back waist portion has a buttocks cover below a lower end of the side joining portion,
at least some of the elastic members is arranged in the buttocks cover, and
a lateral length of the welded portion that is located in the lateral direction nearest to an outer edge portion of the buttocks cover
is longer than
a lateral length of at least one of other welded portion that is located inside the welded portion that is located in the lateral direction nearest to an inner end of the side joining portion.

8. The underpants-shaped absorbent article according to any one of claims 3 to 6, wherein
the back waist portion has a buttocks cover below a lower end of the side joining portion,
a leg elastic member that stretches/contracts along an outer edge portion of the buttocks cover is provided, and
the leg elastic member is attached to the back waist portion with adhesive.

9. The underpants-shaped absorbent article according to any one of claims 3 to 8, wherein
at least one of the skin-side sheet and the non-skin-side sheet is a spunbond nonwoven fabric sheet in which heat-fusing fibers are joined by a plurality of dispersed heat fused portions, and
in the inner end region, either one of the skin-side sheet or the non-skin-side sheet has a portion that overlaps the welded portion and the heat fused portion when seen in the thickness direction.

10. The underpants-shaped absorbent article accrodign to any one of claims 3 to 9, wherein
each of the side joining portions has a plurality of lock welded portions lined up spacing in the vertical direction, and
a depth of the lock welded portion in the thickness direction is deeper than a depth of the welded portion in the thickness direction.

11. The underpants-shaped absorbent article accrodign to claim 10, wherein
a maximum value of a lateral length of the lock welded portions is smaller than a maximum value of a lateral length of the welded portion that is provided in the inner end region.

12. The underpants-shaped absorbent article accrodign to any one of claims 1 to 11, wherein
the waist portion includes:
a first elastic member that is capable of stretching and contracting in the lateral direction, and
a second elastic member that is located adjacent to the first elastic member in the vertical direction,
a plurality of wrinkles along vertical direction are formed by protruding deforming of a sheet member in the thickness direction,
the sheet member being one that constitutes the waist portion, and
at least some of the plurality of wrinkles are continuous between the first elastic member and the second elastic member in the vertical direction.

13. The underpants-shaped absorbent article accrodign to any one of claims 1 to 12, wherein
the absorbent main body includes a liquid-absorbent absorbent core,
the waist portion that is in the stretched state has:
absorbent-body side regions that are located below the upper end of the absorbent main body in the vertical direction and within a range of 20 mm outward from both ends of the absorbent core in the lateral direction; and
leg-circumferential regions that are located below the upper end of the absorbent main body in the vertical direction and are respectively sandwiched between corresponding pairs of the absorbent-body side region and the side joining portion in the lateral direction, and
concerning a difference between a lateral length of the absorbent-body side region in the stretched state and a lateral length of the absorbent-body side region in the natural state,
concerning a difference between a lateral length of the leg-circumferential region in the stretched state and a lateral length of the leg-circumferential region in the natural state,
a value obtained by dividing the difference of the absorbent-body side region by the lateral length of the absorbent-body side region in the stretched state
is smaller than
a value obtained by dividing the difference of the leg-circumferential region by the lateral length of the leg-circumferential region in the stretched state.

14. An underpants-shaped absorbent article having a vertical direction and a lateral direction that intersect with each other, the underpants-shaped absorbent article comprising:
an absorbent main body having a liquid-absorbent absorbent core;
a waist portion in which a front waist portion and a back waist portion are joined to each other by a pair of side joining portions in two ends in the lateral direction;
the waist portion having a stretchability in the lateral direction, in at least a portion located! between the pair of side joining portions,
the waist portion that is in a stretched state having:
an absorbent-body side region that is located below an upper end of the absorbent main body in the vertical direction and within a range of 20 mm outward from two ends of the absorbent core in the lateral direction, and
a leg-circumferential region that is located below the upper end of the absorbent main body in the vertical direction and is sandwiched between the absorbent-body side region and the side joining portion in the lateral direction,
concerning a difference between a lateral length of the absorbent-body side region in the stretched state and a lateral length of the absorbent-body side region in a natural state,
concerning a difference between a lateral length of the leg-circumferential region in the stretched state and a lateral length of the leg-circumferential region in the natural state,
a value obtained by dividing the difference of the absorbent-body side region by the lateral length of the absorbent-body side region in the stretched state
being smaller than
a value obtained by dividing the difference of the leg-circumferential region by the lateral length of the leg-circumferential region in the stretched state.
